# EUROPEAN PATENT SPECIFICATION

(11) **EP 2 798 088 B1**
(45) Date of publication and mention of the grant of the patent: **06.06.2018**
(21) Application number: 12863367.4
(22) Date of filing: 27.12.2012
(51) Int. Cl.: C12Q 1/6886

(54) **DETECTION OF ADENOMAS OF THE COLON OR RECTUM**
NACHWEIS VON ADENOMEN DES DARMS ODER DES REKTUMS
DÉTECTION D'ADÉNOMES DU CÔLON OU DU RECTUM

(30) Priority: 27.12.2011 US 201161580595 P
(43) Date of publication of application: 05.11.2014
(73) Proprietor: Region Syddanmark, 7100 Vejle (DK)
(72) Inventor: KØLVRAA, Steen, DK-8541 Skødstrup (DK); RØNLUND, Karina, DK-7080 Børkop (DK); CRÜGER, Dorthe, DK-8700 Horsens (DK); MORTON, Dion, Birmingham B17 0AN (GB)
(74) Representative: Høiberg P/S
(86) International application number: PCT/DK2012/050510
(87) International publication number: WO 2013/097868

(56) References cited:
- WO-A2-2006/128140
- WO-A2-2009/100161
- WO-A2-2011/126768
- WO-A2-2011/126768
- WO-A2-2012/174256
- LANLAN SHEN ET AL: "Genome-wide profiling of DNA methylation reveals a class of normally methylated CpG island promoters", PLOS GENETICS, vol. preprint, no. 2007, 1 January 2005 (2005-01-01), page e181, XP055009998, ISSN: 1553-7390, DOI: 10.1371/journal.pgen.0030181.eor
- HESSON L.B. ET AL.: 'CpG island promoter hypermethylation of a novel Ras-effector gene RASSF2A is an early event in colon carcinogenesis and correlates inversely with K-ras mutations' ONCOGENE vol. 24, 2005, pages 3987 - 3994, XP003013793
- PERETTI T. ET AL.: 'Heparanase-2, syndecan-1, and extracellular matrix remodeling in colorectal carcinoma' EUROPEAN JOURNAL OF GASTROENTEROLOGY & HEPATOLOGY vol. 20, no. 8, 2008, pages 756 - 765, XP008174294
- ZHANG L. ET AL.: 'Genome-Wide Analysis of Histone H3 Lysine 4 Trimethylation in Peripheral Blood Mononuclear Cells of Minimal Change Nephrotic Syndrome Patients' AMERICAN JOURNAL OF NEPHROLOGY vol. 30, 2009, pages 505 - 513, XP055074445

## Description

### Field of invention

The present invention lies in the field of detection of hyperproliferative diseases. In particular, the invention relates to methods of early detection of hyperproliferative diseases of the colon, for example detection of adenomas of the colon.

### Background of invention

### Colorectal cancer

### Background on colorectal cancer and colorectal adenoma

Colorectal cancer is the third most common malignancy worldwide. In 2008 more than 1.2 million new colorectal cancers were diagnosed and 608.700 died of the disease. Lifetime risk of colorectal cancer is approximately 6% and of colorectal adenoma approximately 50%. The risk rises with age, especially after the age of 60. Most colorectal cancers appear in the rectum (38%) or sigmoid colon (29%).

Colorectal cancers may shed blood and other tissue components that can be detected in the feces long before the development of clinical symptoms. This has led to a search for stool analyses that can allow early detection of cancer, i.e. adenomas. The most common method has been the detection of blood in feces. Faecal Occult Blood testing (FOBT) is a non-invasive and safe test for colorectal cancer. It has a low specificity for colorectal cancer (10% of positive tests) and 50% of "positives" subsequently have a normal colonoscopy. FOBT only identifies 10% of pre-symptomatic adenomas. Despite this several randomized studies has shown that FOBT can reduce the mortality of colorectal cancer by 15-33%. More than 17 countries in Europe has adopted the fecal occult blood test (FOBT) into their national population screening program (© World Gastroenterology Organisation, 2007 Colorectal cancer screening).

The main problem with FOBT is that the sensitivity only is 50-60% for one-time use. The sensitivity increases to 90% when used every 1 - 2 years. Low sensitivity leads to a high number of false-negative results. This can explain interval cancers which appear in periods between biennial screenings.

The specificity is also very low resulting in many false-positive, and these patients will be subjected to unnecessary further investigations, usually colonoscopy. In a Danish pilot project 91.759 patients between 50 and 74 years of age were invited to participate in screening for colorectal cancer using FOBT. 46.693 FOBT was analyzed. 1.037 patients had a positive test. Then colonoscopy detected 79 colorectal cancers and 381 adenomas. The remaining 577 patients were false-positive. Within the 21 months of the study, 23 patients, that have had a negative FOBT, were diagnosed with colorectal cancer, with an interval of 17 to 533 days after having a negative FOBT.

In many countries FOBT is being replaced by fecal immunochemical tests (FIT or IFOBT), which detect hemoglobin using sensitive and specific techniques. These new tests are more expensive and not yet tested in large scale studies. The optimum cut-off point for sensitivity in the immunochemical tests remains to be validated. The FOBT and IFOBT obviate the need for dietary restrictions. The user-friendliness of the tests varies; some are more user-friendly and have very good compliance.

As evidenced above there is an unmet need for a method of detecting colorectal cancer and preferably colorectal adenomas with high specificity and sensitivity.

WO 2011/126768 discloses different methylation markers for colon adenoma. Shen et al. (PLoS Genetics; October 2007; Volume 3, Issue 10; e181) discloses promoter methylation in the OPLAH gene, without however, teaching an association to colon adenoma.

### Summary of invention

The present invention discloses a method for detecting colorectal adenomas with high specificity and sensitivity. It has been established that both 'normal' mucosa and tumour cells are shed into the bowel and then passed with the stool. Identification of abnormal DNA in stool samples provides a method for early detection of colorectal adenoma. Analysis of stool-derived DNA offers the potential for a safe, non-invasive, test that is specific for colorectal adenomas and could be used on its own as well as being possible to use to complement current screening approaches.

Aberrant DNA methylation patterns have been associated with a large number of human malignancies and found in two distinct forms: hypermethylation and hypomethylation compared to normal tissue. Methylation of the DNA is an epigenetic change that occurs at clustered CpG dinucleotides, or CpG rich regions, which contain a higher frequency of cytosine and guanine nucleotide bases than the remainder of the genome. It is now established that methylation of CpG rich regions in the promoter region of tumour suppressor genes results in transcriptional inactivation of the affected gene and methylation is thus implicated in both benign and malignant tumour formation.

The present invention identifies pre-malignant changes in the mucosa of the colon and rectum based on hypermethylation of the DNA and interestingly thereby provides a method for diagnosing adenomas of the colon, preferably even in a non-invasive manner e.g. in stool samples. The FOBT can detect cancers that are bleeding into the lumen, but bleeding is a late symptom and the test is not designed to detect early changes and therefore prevent progression into cancer. By detecting early changes, adenomas could easily be removed during a colonoscopy and the patient would never develop cancer. The present invention may in one embodiment be used alone and may in another embodiment be used as a supplement to the FOBT that we know today, and it will add an extra dimension in detecting more adenomas than the case is today. Most likely it will also increase the number of adenomas found.

In one embodiment the present disclosure relates to a method of determining the methylation status of at least one CpG within a CpG rich region, wherein said CpG rich region is selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 or a sequence at least 90% identical thereto, and wherein the method comprises the step of determining the methylation status of at least one CpG within said CpG rich region in genomic DNA of a sample of an individual at risk of acquiring an adenoma of the colon, and wherein methylation of said at least one CpG is indicative of the presence of an adenoma of the colon in said individual.

### Description of Drawings

Figure 1: Diagram indicating criteria for defining group A, B and C."
Figure 2: Genes contained in group A, B and C

### Detailed description of the invention

### Definitions

The term "methylation" or "DNA methylation" as used herein refers to the addition of a methyl group to the 5 position of the cytosine pyrimidine ring. The methylation reaction involves flipping a target cytosine out of an intact double helix thereby allowing the transfer of a methyl group from S-adenosylmethionine in a cleft of the enzyme DNA (cystosine-5)-methyltransferase to form 5-methylcytosine (5-mCyt).

The term "hypermethylation" as used herein refers to an increase in the methylation of cytosine residues in a sequence of DNA when compared to the same sequence of DNA with no methylation. In the present disclosure a sequence of DNA will be considered hypermethylated when the methylation of said sequence is greater than the mean methylation in the same normal DNA sequence. The increase in methylation from normal tissue to pre-malignant adenomas should be as high as possible. The difference in methylation between the two samples should be highly significant (P < 0.001, two-tailed Mann-Whitney t-test. In the present disclosure the sequence of DNA which is evaluated for methylation is preferably a CpG rich region.

The term "expression level" as used herein refers to the level of a given RNA transcript, or protein of in a given sample, for example in a tissue sample. The level of protein can be determined by for example immunohistochemistry or other methods using antibodies that specifically recognize said protein or fragment thereof. The level of RNA transcript is determined by for example quantitative RT-PCR with normalisation to at least one relevant other gene.

"CpG rich regions" are regions of DNA where a cytosine nucleotide occurs next to a guanine nucleotide in the linear sequence of bases along its length. "CpG" is shorthand for "-C-phosphate-G-", that is, cytosine and guanine separated by only one phosphate; phosphate links any two nucleosides together in DNA. The "CpG" notation is used to distinguish this linear sequence from the CG base-pairing of cytosine and guanine. A CpG islands of the present disclosure is a region of at least 200 bp and said region is typically 300-3,000 base pairs in length. The GC percentage of said region is typically greater than 50% and with an observed-to-expected CpG ratio greater than 0.6. A CpG rich region according to the disclosure preferably consists of a continuous stretch in the range of 700 to 11000 base pairs, such as in the range of 700 to 5700 base pairs, preferably in the range of 700 to 2000 base pairs, wherein at least 50% is C or G, comprising one or more CpG island(s).
In one embodiment of the present disclosure the CpG rich region is a continuous stretch of at the most 10000 base pairs, preferably at the most 9000 base pairs, preferably at the most 8000 base pairs, preferably at the most 7000 base pairs, preferably at the most 6000 base pairs, preferably at the most 5000 base pairs, preferably at the most 4000 base pairs, preferably at the most 3000 base pairs, preferably at the most 2000 base pairs, preferably at the most 1000 base pairs, preferably at the most 500 base pairs, preferably at the most 300 base pairs.

"Methylation of a CpG" denotes methylation of the 5 position of the pyrimidine ring of the cytosine of a CpG dinucleotide to form 5-methylcytosine. Thus a methylated CpG will comprise a 5-methylcytosine of the formula: 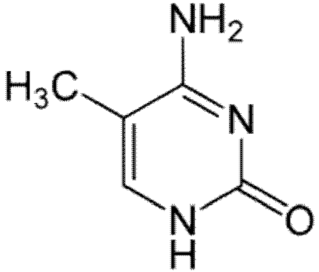 and a G connected by a DNA backbone.

A methylated CpG could accordingly be described by the formula:

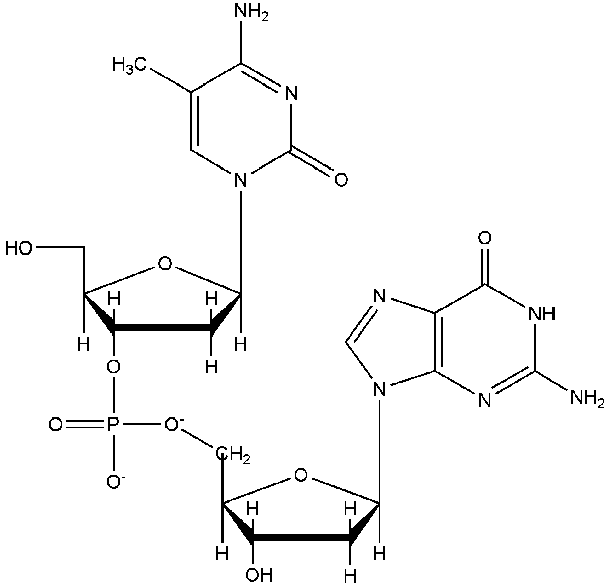

"Adenoma of the colon" is a pre-malignant, benign tumour of the colon. Adenomas may develop into malignant tumours.

### Adenomas and colon cancer

The present invention discloses the use of the methylation status of CpG rich regions linked to different genes as markers for adenomas. Said invention may be used in a method for determining an individual at risk of contracting colon cancer and/or adenomas. Colorectal cancer is the third most common malignancy worldwide. Risk factors for colorectal cancer include overweight and obesity, type 2 diabetes and high consumption of alcohol. Smoking, physical inactivity and meat consumption are also considered as risk factors. The most common symptoms of colorectal cancer are stomach ache, diarrhea, constipation, changes in stool pattern and in some cases blood in stool. But there can also be other symptoms. Today 60% of the patients with colorectal cancer are diagnosed with advanced disease, mainly because the clinical symptoms only appear late in the cancer progression.

Based on evidence from epidemiological and pathological studies, most sporadic colorectal cancers are thought to develop from benign adenomas. Adenomas can occur anywhere in the colon or rectum and generally represent masses that protrude into the gut lumen (polyps). More rarely adenomas can be flat or depressed. The adenoma-carcinoma model (Morson BC, 1978, "The Pathogenesis of Colorectal Cancer. Later in the 1990es", Vogelstein B et al.,1988, "Genetic alterations during colorectal-tumor development", N Engl J Med, 319:525-332) of colorectal carcinogenesis has generally been accepted for decades, but only a small percentage of adenomas progress to carcinomas. Progression is associated with severe dysplasia, patient age and size of the adenoma.

By determining the methylation status of certain CpG rich regions the present invention may be used for identifying the presence of adenomas at an early stage and/or to determine the risk of developing adenomas. Hypermethylation of a given CpG rich region is indicative of the presence of adenomas and/or an increased risk of developing adenomas. The adenomas to be identified using the present invention may for example be Tubulovillous Adenoma (TVA), tubular adenoma and villous adenoma.The determination of the methylation status of a given CpG rich region is described herein below and evaluating whether a given region is hypermethylated is also described.

### Samples

The sample that is used in the methods of the present invention may be in a form suitable to allow analysis by the skilled artisan. The samples to be used with the present invention may be selected from the group consisting of a tissue sample, or from body fluid samples.

In a preferred embodiment of the present invention the sample is body fluid sample selected from the group consisting of blood, plasma, serum, and faecal samples. In a preferred embodiment the sample is a faecal sample.

In another preferred embodiment of the present invention, the sample is a tissue sample, such as a biopsy of the tissue, or a superficial sample scraped from the tissue. In another embodiment the sample may be prepared by forming a suspension of cells made from the tissue. The sample may, however, also be an extract obtained from the tissue or obtained from a cell suspension made from the tissue.

In a preferred embodiment the sample used in the methods of the present invention is a biopsy of the colon lining or resected colon tissue.

Working with tumor material in general requires biopsies or body fluids suspected to comprise relevant cells. Working with RNA in general requires freshly frozen or immediately processed biopsies, or chemical pretreatment of the biopsy. Apart from the cancer tissue, biopsies do normally contain many different cell types, such as cells present in the blood, connective and muscle tissue, endothelium etc. In the case of DNA studies, microdissection or laser capture are methods of choice, however the time-dependent degradation of RNA may make it difficult to perform manipulation of the tissue for more than a few minutes. The sample may be fresh or frozen, or treated with chemicals.

### Determining the methylation status and the methylation incidence of CpG rich regions

The present disclosure discloses methods of detecting the presence of adenomas and/or the risk of developing adenomas by determining the methylation status of a CpG rich region present in a sequence of DNA isolated for a sample (described in the section "Samples"). Determining the methylation status and/or methylation incidence in the methods according to the disclosure may be done using any one of the methods mentioned herein below or using any other suitable method known to the skilled person.

A number of current methodologies for methylation studies are well known to the skilled person. Sequencing of bisulphite-treated DNA is the gold standard for methylation studies as it reveals directly the status of each CpG dinucleotide. Thus, in a preferred embodiment of the invention the methylation status of one or more CpG rich regions linked to either of the genes described in the section "Gene of interest" may be determined by sequencing of bisulphite-treated DNA. Bisulphate-based methylation genomic sequencing is capable of detecting every methylated cytosine on both strands of any target sequence, using DNA isolated from a small number of cells, though of course more cells may be employed. In this method, sodium bisulphite is used to convert cytosine residues to uracil residues in single-stranded DNA, under conditions whereby 5-methylcytosine remains non-reactive. The converted DNA is amplified with specific primers and sequenced. All the cytosine residues remaining in the sequence represent previously methylated cytosines in the genome. This method may utilize defined procedures that maximize the efficiency of denaturation, bisulphite conversion and amplification, to permit methylation mapping of single genes from small amounts of genomic DNA (see Grigg G, Clark S. (June 1994) "Sequencing 5-methylcytosine residues in genomic DNA" Bioessays.16(6):431-6).

Methylation status of the CpG rich region can also be measured quantitatively by pyrosequencing of bisulfite converted DNA following PCR amplification. Pyrosequencing is a sequencing-by-synthesis method that relies on the sequential addition and incorporation of nucleotides in a primer-directed polymerase extension. Only one of the four nucleotides is present at any time in the reaction vessel, and only if the added nucleotide is complementary to the template DNA will it be incorporated by a DNA polymerase. This event is monitored in real time and hence can be used to quantitate the ratio between methylated and unmethylated CpG dinucleotides. The pyrosequencing technology makes use of the release of PPᵢ molecules during the iterative incorporation of unmodified nucleotides that are quantitatively converted into a bioluminometric signal (Tost, J. & Gut, I.G. DNA methylation analysis by pyrosequencing, Nature Protocols 2, - 2265 - 2275 (2007)). Pyrosequencing may be used for determination of the methylation status of one or more CpG rich regions linked to either of the genes described in the section "Gene of interest".

According to the present invention the methylation incidence may be determined using methylation specific PCR. Methylation specific PCR (MSP) is one of the most widely used assays for the sensitive detection of methylation. US patent 5,786,146 discloses a method of methylation specific PCR (MSP) for identifying DNA methylation patterns in a CpG containing nucleic acid, which is useful for use with the present invention. The methylation status of one or more CpG rich regions linked to either of the genes described in the section "Gene of interest" may be determined using this method. The skilled person would know how to adapt the method for use in the present invention. The method uses agents to modify unmethylated cytosine in the nucleic acid. Prior to amplification, the DNA is treated with sodium bisulphite to convert all unmethylated cytosines to uracils. The bisulphite reaction effectively converts methylation information into sequence difference. CpG specific oligonucleotide primers are used to distinguish between modified methylated and unmethylated nucleic acid. The identification of the methylated nucleic acid is based on the presence or absence of amplification product resulting from the amplification and distinguishing modified methylated and non-methylated nucleic acids. The generated PCR product can for example be visualized on a gel.

An important parameter for the specificity of methylation-specific PCR is determined by primer design. Since modification of DNA by bisulfite destroys strand complementarity, either strand can serve as the template for subsequent PCR amplification and the methylation pattern of each strand can then be determined. It will be appreciated, though, that amplifying a single strand (e.g. sense strand) is preferable in practice. Primers are preferably designed to amplify a region that is 80-250 bp in length, which incorporates a sufficient number of cytosines in the original strand to assure that unmodified DNA does not serve as a template for the primers. In addition, the number and position of cytosines within the CpG dinucleotide determines the specificity of the primers for methylated and unmethylated templates. The primers to be used with the methods of the present invention are preferably between 15 and 25 nucleotides long. One of the primers are identical to a sequence of DNA contained in the CpG rich region linked to either of the genes mentioned in the section "Genes of interest" while the other primer is complementary thereto. The primer is preferably at least 95% identical to a sequence of the CpG rich region, preferably 100% identical. Typically, 1-3 CpG sites are included in each primer and concentrated in the 3' region of each primer preferably, i.e. the end of the primer that is located closest to the annealing site of the DNA polymerase. This provides optimal specificity and minimizes false positives due to mispriming. To facilitate simultaneous analysis of each of the primers of a given gene in the same thermocycler, the length of the primers is adjusted to give nearly equal melting/annealing temperatures.

Methylation status may also be determined using Real-time fluorescent MSP (MethyLight), which is based on real time PCR employing fluorescent probes in conjunction with MSP and allows for a homogeneous reaction which is of higher throughput. Thus methylation status according to the methods of the invention may also be determined using real-time fluorescent MSP. If the probe does not contain CpGs, the reaction is essentially a quantitative version of MSP. However, the fluorescent probe is typically designed to anneal to a site containing one or more CpGs linked to either of the genes described in the section "Gene of interest", and this third oligonucleotide increases the specificity of the assay for completely methylated target strands. The methylation status of one or more CpG rich regions linked to either of the genes described in the section "Gene of interest" may be determined using this method. Because the detection of the amplification occurs in real time, there is no need for a secondary electrophoresis step. Since there is no post PCR manipulation of the sample, the risk of contamination is reduced. The MethyLight probe can be of any format including but not limited to a Taqman probe or a LightCycler hybridization probe pair and if multiple reporter dyes are used, several probes can be performed simultaneously. Real-time fluorescent MSP is the preferred method for determining the methylation incidence according to the present invention.

Methods such as a PCR-based high-resolution melting analysis assay may also be used to determine the methylation status of one or more CpG rich regions linked to either of the genes described in the section "Gene of interest". Methylation-sensitive high-resolution melting (MS-HRM) analysis is one PCR-based technology which can be used for determination of methylation status of the CpG rich region according to the invention and for highly specific and highly sensitive detection of methylation of one or more CpG rich regions linked to either of the genes described in the section "Gene of interest". This method takes advantage of the fact that methylated DNA and unmethylated DNA acquire different sequences after bisulphite treatment, which results in PCR products with markedly different melting profiles/temperature. PCR is used to amplify both methylated and unmethylated sequences in the same reaction, and this method can be optimised to detect methylation levels as low as 0.1%.
The primers to be used with the methods of the present invention are preferably between 15 and 25 nucleotides long. One of the primers are identical to a sequence of DNA contained in the CpG rich region linked to either of the genes mentioned in the section "Genes of interest" while the other primer is complementary thereto. The primer is preferably at least 95% identical to a sequence of the CpG rich region, preferably 100% identical.
MS-HRM also allows estimation of methylation levels by comparison of melting profiles for a test sample to the melting profiles of PCR products derived from standards with known ratios of methylated:unmethylated alleles. MS-HRM analysis protocols are simple and the method is characterized by high reproducibilty (Wojdacz TK, Dobrovic A, Hansen LL. 2008, Nat Protoc. 2008;3(12):1903-8).

For rapid assessment of CpG methylation status of a DNA region the quantitative methylation density assay may be used as previously described by Galm et al. (2002) Genome Res. 12, 153-7. After bisulfite modification of genomic DNA, the region of interest is PCR amplified with nested primers. Primers should be directed at one or more CpG rich regions linked to either of the genes described in the section "Gene of interest". One of the primers used should preferably be equal to a sequence of the CpG rich region of interest (see section of "gene of interest") and the other primer should be complementary there to. In one embodiment the primer is at least 95% identical to said sequence, preferably 100% identical. PCR products are purified and DNA amount is determined. A predetermined amount of DNA is incubated with ³H-SAM (TRK581Bioscience, Amersham) and Sssl methyltransferase for methylation quantification. Once reactions are terminated products are purified from the in-vitro methylation mixture. 20% of the eluant volume is counted in ³H counter. Normalizing radioactivity DNA of each sample is measured again and the count is normalized to the DNA amount.

In a preferred embodiment of the invention, multiplex ligation-dependent probe amplification (MLPA) may be used to determine the methylation status of CpG rich regions using the methylation specific MLPA protocol (MS-MLPA). MS-MLPA can be used for DNA and mRNA profiling of both blood and tissue samples. MS-MLPA is a semi-quantitative methylation detection method and is a variant of standard MLPA. In MLPA a mixture of unique MLPA probes are added to the nucleic acid sample. MLPA probes consist of two separate oligonucleotides, each containing one of the PCR primer sequences. The primers to be used with the methods of the present invention are preferably between 15 and 25 nucleotides long. One of the primers is preferably identical to a sequence of DNA contained in the CpG rich region linked to either of the genes mentioned in the section "Genes of interest" while the other primer is complementary thereto. The primer is preferably at least 95% identical to a sequence of the CpG rich region, preferably 100% identical.
The MS-MLPA protocol is very similar to the standard MLPA method, except that each MS-MLPA reaction generates two samples: one undigested sample for copy number detection and one digested sample for methylation detection. The MS-MLPA procedure can in general be divided in five steps: 1. DNA denaturation and hybridisation of MLPA probes; 2. ligation and digestion; 3. PCR; 4. separation of amplification products by capillary electrophoresis and 5. data analysis.
MS-MLPA probes for methylation detection resemble other MLPA probes, except that their target sequence contains the restriction site of the methylation-sensitive endonuclease Hhal. After hybridisation, the reaction is split into two tubes: one tube is processed as a standard MLPA reaction, providing information on copy number changes. The other is incubated with the Hhal endonuclease meanwhile hybridized probes are ligated. Hybrids of probes and unmethylated sample DNA are digested by the Hhal enzyme. Digested probes cannot be amplified exponentially during PCR and hence will not produce a signal during capillary electrophoresis. In contrast, if the sample DNA is methylated, the DNA-probe hybrids are protected against Hhal digestion and the ligated probes will generate a peak.

In yet another embodiment, immunoprecipitation of methylated sequences can be used to isolate sequence-specific methylated nucleotide fragments. Briefly, genomic DNA is sonicated to yield fragments of 200-300 bp. The DNA is then denatured, precleaned with a protein A Fast FlowSepharose) and further incubated with a 5-methylcytidine monoclonal antibody. The complex may be purified using protein A Sepharose and subsequently washed. The immunoprecipitated samples are then analyzed using specific PCR primers or microarray technology.

In yet another embodiment bisulfit-treated DNA is analysed on a high density oligo array where there for each potentially methylated C to be analyzed on the array is both an oligo specific for a C and an oligo specific for an U. Perfect annealing of bisulfit-treated DNA to oligos is detected by polymerase-catalyzed subsequent addition of a fluorescent nucleotide. By analyzing all oligo-spots the degree of methylation on all potentially methylated C's can be determined.
Oligos should be directed at one or more CpG rich regions linked to either of the genes described in the section "Gene of interest". Each potentially methylated C of that CpG rich region should preferably be matched with an oligo. In one embodiment oligos are directed to at least 95% of the potentially methylated C's in said sequence, preferably 100%.

Methylation status of a CpG rich region may also be determined by targeted resequencing of bisulfite converted DNA (or PCR amplicons hereof) using massively parallel sequencing technologies (aka next-generation sequencing) and possibly future sequencing platforms able to sequence individual DNA molecules. This would allow digital quantification of the ratio between methylated and unmethylated CpG dinucleotide(s). Next-generation sequencing platforms are characterized by the ability to process millions of sequence reads in parallel rather than 96 at a time, as typically seen for capillary-based sequencing. The workflow to produce next-generation sequence-ready libraries is straightforward; DNA fragments are prepared for sequencing by ligating specific adaptor oligos to both ends of each DNA fragment. Importantly, relatively little input DNA (a few micrograms at most) is needed to produce a library. Currently available platforms for next-generation sequencing produce shorter read lengths (35-250 bp, depending on the platform), but longer reads will also be possible. (Mardis, E.R. The impact of next-generation sequencing technology on genetics, Trends Genet. 2008 Mar;24(3):133-41). The method may be used for determination of methylation status of one or more CpG rich regions linked to either of the genes described in the section "Gene of interest".

In either of the PCR based methods mentioned herein above the primer and/or probe may comprise or even consist of DNA analogous, e.g. the TINA molecules as described in WO2009/112032, incorporated herein by reference.

One PCR-based method is the ligase chain reaction (LCR). LCR is another method of DNA amplification which may be used in combination with the relevant methods mentioned herein above. The skilled person will know in which of the methods mentioned herein above PCR may be exchanged with LCR. A key advantage of LCR is greater specificity as compared to PCR.

### Gene of interest

The present disclosure relates to the determination of the methylation status of CpG rich regions linked to specific gene of interest. CpG rich regions are defined in the section "Definitions" herein above.
A CpG rich region of the present disclosure is preferably selected from the group consisting of SEQ ID NO.1, SEQ ID NO.2, SEQ ID NO.3, SEQ ID NO.4, SEQ ID NO. 5, SEQ ID NO.6, SEQ ID NO.7, SEQ ID NO.8, SEQ ID NO.9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO. 39, SEQ ID NO. 40, SEQ ID NO. 41, SEQ ID NO. 42, SEQ ID NO. 43, SEQ ID NO. 44, and SEQ ID NO. 45.

In another embodiment the CpG rich region of the present disclosure is preferably selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11

In another embodiment the CpG rich region of the present disclosure is preferably selected from the group consisting of SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91.

In another embodiment the CpG rich region of the present disclosure is preferably selected from the group consisting of SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, and SEQ ID NO: 44.

A CpG rich region of the present invention is preferably a region wherein there is a high difference in the methylation status between adenomas and normal tissues and where the methylation status in general is low in the normal tissue. The methylation status may be determined using any of the methods disclosed herein above in the section "Determining the methylation status and the methylation incidence of CpG rich regions".

When determining the methylation status of a CpG rich region according to the invention in one embodiment the methylation status is determined of at least 2 CpG's in either the same CpG rich region or preferably in different CpG rich regions.

In one embodiment methylation status of at least 2 CpG's within one CpG rich region is determined, wherein methylation of both of said CpG's is indicative of the presence of an adenoma of the colon.
In another embodiment methylation status of at least 4 CpG's within one CpG rich region is determined, wherein methylation of at least 2 of said CpG's is indicative of the presence of an adenoma of the colon.
In another embodiment methylation status of at least 2 CpG's within at least 2 different CpG rich regions are determined, and wherein methylation of at least 2 of said CpG's is indicative of the presence of an adenoma of the colon.
In another embodiment methylation status of at least 4 CpG's within at least 4 different CpG rich regions are determined, and wherein methylation of at least 2 of said CpG's is indicative of the presence of an adenoma of the colon.
In another embodiment methylation status of at least 6 CpG's within at least 6 different CpG rich regions are determined, and wherein methylation of at least 4 of said CpG's is indicative of the presence of an adenoma of the colon.
In another embodiment methylation status of at least 8 CpG's within at least 8 different CpG rich regions are determined, and wherein methylation of at least 6 of said CpG's is indicative of the presence of an adenoma of the colon.
In another embodiment methylation status of at least 10 CpG's within at least 10 different CpG rich regions are determined, and wherein methylation of at least 8 of said CpG's is indicative of the presence of an adenoma of the colon.
In another embodiment methylation status of at least 12 CpG's within at least 12 different CpG rich regions are determined, and wherein methylation of at least 10 of said CpG's is indicative of the presence of an adenoma of the colon.
In another embodiment methylation status of at least 14 CpG's within at least 14 different CpG rich regions are determined, and wherein methylation of at least 12 of said CpG's is indicative of the presence of an adenoma of the colon.

In another embodiment methylation status of at least 16 CpG's within at least 16 different CpG rich regions are determined, and wherein methylation of at least 14 of said CpG's is indicative of the presence of an adenoma of the colon.
In another embodiment methylation status of at least 18 CpG's within at least 18 different CpG rich regions are determined, and wherein methylation of at least 16 of said CpG's is indicative of the presence of an adenoma of the colon.
In another embodiment methylation status of at least 20 CpG's within at least 20 different CpG rich regions are determined, and wherein methylation of at least 18 of said CpG's is indicative of the presence of an adenoma of the colon.

A preferred CpG rich region of the present disclosure is SEQ ID NO: 1. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 2. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 3. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 4. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 5. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 6. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 7. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 8. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 9. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 10. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 11. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 12. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 13. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 14. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 15. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 16. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 17. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 18. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 19. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 20. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 21. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 22. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 23. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 24. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 25. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 26. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 27. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 28. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 29. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 30. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 31. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 32. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 33. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 34. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 35. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 36. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 37. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 38. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 39. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 40. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 41. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 42. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 43. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 44. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 45. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 46. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 47. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 48. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 49. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 50. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 51. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 52. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 53. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 54. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 55. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 56. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 57. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 58. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 59. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 60. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 61. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 62. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 63. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 64. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 65. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 66. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 67. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 68. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 69. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 70. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 71. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 72. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 73. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 74. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 75. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 76. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 77. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 78. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 79. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 80. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 81. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 82. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 83. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 84. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 85. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 86. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 87. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 88. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 89. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

A preferred CpG rich region of the present disclosure is SEQ ID NO: 90. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.
A preferred CpG rich region of the present disclosure is SEQ ID NO: 91. In one embodiment of the present disclosure the methylation status of at least 1, such as 2, more preferably at least 3, such as 4, more preferably at least 5 CpG's present within this CpG rich region is evaluated, and wherein methylation of at least half of the evaluated CpG's is indicative of an adenoma of the colon.

In one embodiment the methylation status of at least one CpG from a CpG rich region selected from the group consisting of SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 is determined in combination with determination of the methylation status of at least one CpG from a CpG rich region selected from the group consisting of SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20.
In another embodiment the methylation status of at least one CpG from a CpG rich region selected from the group consisting of SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 is determined in combination with determination of the methylation status of at least one CpG from a CpG rich region selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11.
In another embodiment the methylation status of at least one CpG from a CpG rich region selected from the group consisting of SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20 is determined in combination with determination of the methylation status of at least one CpG from a CpG rich region selected from the group consisting of of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11.

In one embodiment the methylation status of at least one CpG from a CpG rich region selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 is determined in combination with determination of the methylation status of at least one CpG from a CpG rich region selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:9, SEQ ID NO:12, SEQ ID NO: 15, SEQ ID NO:16, SEQ ID NO:17, SEQ ID NO:18, SEQ ID NO:19, SEQ ID NO:20, SEQ ID NO:21, SEQ ID NO:22, SEQ ID NO: 23, SEQ ID NO:24, SEQ ID NO:27, SEQ ID NO:28, SEQ ID NO: 29, SEQ ID NO: 30, SEQ ID NO:31, SEQ ID NO:32, SEQ ID NO:34, SEQ ID NO:35, SEQ ID NO:37, SEQ ID NO:38, SEQ ID NO:40, SEQ ID NO:41, SEQ ID NO:42, SEQ ID NO:43, SEQ ID NO:44, SEQ ID NO:45
In one embodiment the methylation status of at least one CpG from a CpG rich region selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 is determined in combination with determination of the methylation status of at least one CpG from a CpG rich region selected from the group consisting of SEQ ID NO:2, SEQ ID NO:3, SEQ ID NO:4, SEQ ID NO:5, SEQ ID NO:6, SEQ ID NO:7, SEQ ID NO:9, SEQ ID NO:12, SEQ ID NO:16, SEQ ID NO:17, SEQ ID NO:18, SEQ ID NO:19, SEQ ID NO:20, SEQ ID NO:21, SEQ ID NO:22, SEQ ID NO:24, SEQ ID NO:27, SEQ ID NO:28, SEQ ID NO:31, SEQ ID NO:32, SEQ ID NO:34, SEQ ID NO:35, SEQ ID NO:37, SEQ ID NO:38, SEQ ID NO:40, SEQ ID NO:41, SEQ ID NO:42, SEQ ID NO:43, SEQ ID NO:44, SEQ ID NO:45 In one embodiment the methylation status of at least one CpG from a CpG rich region selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:9, SEQ ID NO:12, SEQ ID NO: 15, SEQ ID NO:16, SEQ ID NO:17, SEQ ID NO:18, SEQ ID NO:19, SEQ ID NO:20, SEQ ID NO:21, SEQ ID NO:22, SEQ ID NO: 23, SEQ ID NO:24, SEQ ID NO:27, SEQ ID NO:28, SEQ ID NO: 29, SEQ ID NO: 30, SEQ ID NO:31, SEQ ID NO:32, SEQ ID NO:34, SEQ ID NO:35, SEQ ID NO:37, SEQ ID NO:38, SEQ ID NO:40, SEQ ID NO:41, SEQ ID NO:42, SEQ ID NO:43, SEQ ID NO:44, SEQ ID NO:45 is determined in combination with determination of the methylation status of at least one CpG from a CpG rich region selected from the group consisting of SEQ ID NO:2, SEQ ID NO:3, SEQ ID NO:4, SEQ ID NO:5, SEQ ID NO:6, SEQ ID NO:7, SEQ ID NO:9, SEQ ID NO:12, SEQ ID NO:16, SEQ ID NO:17, SEQ ID NO:18, SEQ ID NO:19, SEQ ID NO:20, SEQ ID NO:21, SEQ ID NO:22, SEQ ID NO:24, SEQ ID NO:27, SEQ ID NO:28, SEQ ID NO:31, SEQ ID NO:32, SEQ ID NO:34, SEQ ID NO:35, SEQ ID NO:37, SEQ ID NO:38, SEQ ID NO:40, SEQ ID NO:41, SEQ ID NO:42, SEQ ID NO:43, SEQ ID NO:44, SEQ ID NO:45.

In one embodiment at least two different CpG rich regions are evaluated for the methylation status, preferably at least 3, such as at least 4, more preferably at least 5, such as at least 6, more preferably at least 7, such as at least 8, more preferably at least 9, such as at least 10.

In one embodiment, the determination of the methylation status of at least one CpG in SEQ ID NO: 1 is combined with the determination of the methylation status of at least one CpG present in the CpG rich region selected from the group consisting of: SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpGs, are indicative of an adenoma of the colon.

In one embodiment, the determination of the methylation status of at least one CpG in SEQ ID NO: 8 is combined with the determination of the methylation status of at least one CpG present in the CpG rich region selected from the group consisting of: SEQ ID NO: 1, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpGs, are indicative of an adenoma of the colon.

In one embodiment, the determination of the methylation status of at least one CpG in SEQ ID NO: 10 is combined with the determination of the methylation status of at least one CpG present in the CpG rich region selected from the group consisting of: SEQ ID NO: 1, SEQ ID NO: 8, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 11 is combined with the determination of the methylation status of at least one CpG present in the CpG rich region selected from the group consisting of: SEQ ID NO: 1, SEQ ID NO: 8, SEQ ID NO: 10, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 18 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 22 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 23 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 25 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 32 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 33 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 35 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 36 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 38 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 44 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 46 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 47 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 48 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 49 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 50 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 51 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 52 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 53 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 54 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 55 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 56 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 57 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 58 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 59 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 60 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 61 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 62 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon

In one embodiment, the determination of the methylation status of at least one CpG in SEQ ID NO: 63 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 64 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 65 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 66 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 67 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 68 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 69 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 70 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 71 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 72 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 73 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 74 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 75 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 76 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 77 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 78 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 79 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 80 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 81 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 82 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 83 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 84 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 85 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 86 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 87 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 88 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 89 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 90, and SEQ ID NO: 91. Preferably methylation of at least half, such as of at least two of said CpG is indicative of an adenoma of the colon.

In one embodiment, the determination of the methylation status of at least one CpG in SEQ ID NO: 90 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, and SEQ ID NO: 91. Preferably methylation of at least half, such as of at least two of said CpG is indicative of an adenoma of the colon.

In one embodiment, the determination of the methylation status of at least one CpG in SEQ ID NO: 91 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, and SEQ ID NO: 90. Preferably methylation of at least half, such as of at least two of said CpG is indicative of an adenoma of the colon.

In one embodiment the methylation status of the CpG rich region of SEQ ID NO: 1 is determined by evaluating the methylation status of at least one CpG within the region of SEQ ID NO: 1, more preferably within the region +/- 200 base pairs from position 172 and/or within the region +/- 200 base pairs from position 284 in SEQ ID NO: 1, even more preferably within the region +/- 100 base pairs from position 172 and/or within the region +/- 100 base from position 284 in SEQ ID NO: 1, even more preferably within the region +/- 50 base pairs from position 172 and/or within the region +/- 50 base pairs from position 284 in SEQ ID NO: 1 for the presence of methylated CpGs. In a most preferred embodiment the methylation status of the CpG of position 172 and/or position 284 of SEQ ID NO: 1 is determined.
If the at least one CpG within the region of SEQ ID NO: 1 is methylated, said methylation is indicative of the presence of an adenoma of the colon.

In one embodiment the methylation status of the CpG rich region of SEQ ID NO: 8 is determined by evaluating the methylation status of at least one CpG within the region of SEQ ID NO: 8, more preferably within the region +/- 200 base pairs from position 226 and/or within the region +/- 200 base pairs from position 831 in SEQ ID NO: 8, even more preferably within the region +/- 100 base pairs from position 226 and/or within the region +/- 100 base pairs from position 831 in SEQ ID NO: 8, even more preferably within the region +/- 50 base pairs from position 226 and/or within the region +/- 50 base pairs from position 831 in SEQ ID NO: 8 for the presence of methylated CpGs. In a most preferred embodiment the methylation status of the CpG of position 226 and/or position 831 of SEQ ID NO: 8 is determined.
If the at least one CpG within the region of SEQ ID NO: 8 is methylated, said methylation is indicative of the presence of an adenoma of the colon.

In one embodiment the methylation status of the CpG rich region of SEQ ID NO: 10 is determined by evaluating the methylation status of at least one CpG within the region of SEQ ID NO: 10, more preferably within the region +/- 200 base pairs from position 101 and/or within the region +/-200 base pairs from position 643 in SEQ ID NO: 10, even more preferably within the region +/- 100 base pairs from position 101 and/or within the region +/- 100 base pairs from position 643 in SEQ ID NO: 10, even more preferably within the region +/- 50 base pairs from position 101 and/or within the region +/- 50 base pairs from position 643 in SEQ ID NO: 10 for the presence of methylated CpGs. In a most preferred embodiment the methylation status of the CpG of position 101 and/or position 643 of SEQ ID NO: 10 is determined.
If the at least one CpG within the region of SEQ ID NO: 10 is methylated, said methylation is indicative of the presence of an adenoma of the colon.

In one embodiment the methylation status of the CpG rich region of SEQ ID NO: 11 is determined by evaluating the methylation status of at least one CpG within the region of SEQ ID NO: 11, more preferably within the region +/- 200 base pairs from position 612 and/or within the region +/- 200 base pairs from position 668 and/or within the region +/- 200 base pairs from position 835 in SEQ ID NO: 11, even more preferably within the region +/- 100 base pairs from position 612 and/or within the region +/- 100 base pairs from position 668 and/or within the region +/- 100 base pairs from position 835 in SEQ ID NO: 11, even more preferably within the region +/- 50 base pairs from position 612 and/or within the region +/- 50 base pairs from position 668 and/or within the region +/- 50 base pairs from position 835 in SEQ ID NO: 11 for the presence of methylated CpGs. In a most preferred embodiment the methylation status of the CpG of position 612 and/or position 668 and/or position 835 of SEQ ID NO: 11 is determined.
If the at least one CpG within the region of SEQ ID NO: 11 is methylated, said methylation is indicative of the presence of an adenoma of the colon.

In one embodiment the methylation status of the CpG rich region of SEQ ID NO: 18 is determined by evaluating the methylation status of at least one CpG within the region of SEQ ID NO: 18, more preferably within the region +/- 200 base pairs from position 231 and/or within the region +/- 200 base pairs from position 1072 and/or within the region +/- 200 base pairs from position 1074 and/or within the region +/- 200 base pairs from position 1086 and/or within the region +/- 200 base pairs from position 1097 and/or within the region +/- 200 base pairs from position 1110 and/or within the region +/- 200 base pairs from position 1115 and/or within the region +/- 200 base pairs from position 1176 in SEQ ID NO: 18, even more preferably within the region +/- 100 base pairs from position 231 and/or within the region +/- 100 base pairs from position 1072 and/or within the region +/- 100 base pairs from position 1074 and/or within the region +/- 100 base pairs from position 1086 and/or within the region +/- 100 base pairs from position 1097 and/or within the region +/- 100 base pairs from position 1110 and/or within the region +/- 100 base pairs from position 1115 and/or within the region +/- 100 base pairs from position 1176 in SEQ ID NO: 18, even more preferably within the region +/- 50 base pairs from position 231 and/or within the region +/- 50 base pairs from position 1072 and/or within the region +/- 50 base pairs from position 1074 and/or within the region +/- 50 base pairs from position 1086 and/or within the region +/- 50 base pairs from position 1097 and/or within the region +/- 50 base pairs from position 1110 and/or within the region +/- 50 base pairs from position 1115 and/or within the region +/- 50 base pairs from position 1176 in SEQ ID NO: 18 for the presence of methylated CpGs. In a most preferred embodiment the methylation status of the CpG of position 231 and/or position 1072 and/or position 1074 and/or position 1086 and/or position 1097 and/or position 1110 and/or position 1115 and/or position 1176 of SEQ ID NO: 18 is determined.
If the at least one CpG within the region of SEQ ID NO: 18 is methylated, said methylation is indicative of the presence of an adenoma of the colon.

In one embodiment the methylation status of the CpG rich region of SEQ ID NO: 22 is determined by evaluating the methylation status of at least one CpG within the region of SEQ ID NO: 22, more preferably within the region +/- 200 base pairs from position 292 and/or within the region +/- 200 base pairs from position 603 and/or within the region +/- 200 base pairs from position 612 and/or within the region +/- 200 base pairs from position 694 and/or within the region +/- 200 base pairs from position 1051 and/or within the region +/- 200 base pairs from position 1330 in SEQ ID NO: 22, even more preferably within the region +/- 100 base pairs from position 292 and/or within the region +/- 100 base pairs from position 603 and/or within the region +/- 100 base pairs from position 612 and/or within the region +/- 100 base pairs from position 694 and/or within the region +/- 100 base pairs from position 1051 and/or within the region +/- 100 base pairs from position 1330 in SEQ ID NO: 22, even more preferably within the region +/- 50 base pairs from position 292 and/or within the region +/- 50 base pairs from position 603 and/or within the region +/- 50 base pairs from position 612 and/or within the region +/- 50 base pairs from position 694 and/or within the region +/- 50 base pairs from position 1051 and/or within the region +/- 50 base pairs from position 1330 in SEQ ID NO: 22 for the presence of methylated CpGs. In a most preferred embodiment the methylation status of the CpG of position 292 and/or position 603 and/or position 612 and/or position 694 and/or position 1051 and/or position 1330 of SEQ ID NO: 22 is determined.
If the at least one CpG within the region of SEQ ID NO: 22 is methylated, said methylation is indicative of the presence of an adenoma of the colon.

In one embodiment the methylation status of the CpG rich region of SEQ ID NO: 23 is determined by evaluating the methylation status of at least one CpG within the region of SEQ ID NO: 23, more preferably within the region +/- 200 base pairs from position 393 and/or within the region +/- 200 base pairs from position 395 and/or within the region +/- 200 base pairs from position 398 and/or within the region +/- 200 base pairs from position 482 in SEQ ID NO: 23, even more preferably within the region +/- 100 base pairs from position 393 and/or within the region +/- 100 base pairs from position 395 and/or within the region +/- 100 base pairs from position 398 and/or within the region +/- 100 base pairs from position 482 in SEQ ID NO: 23, even more preferably within the region +/- 50 base pairs from position 393 and/or within the region +/- 50 base pairs from position 395 and/or within the region +/- 50 base pairs from position 398 and/or within the region +/- 50 base pairs from position 482 in SEQ ID NO: 23 for the presence of methylated CpGs. In a most preferred embodiment the methylation status of the CpG of position 393 and/or position 395 and/or position 398 and/or position 482 of SEQ ID NO: 23 is determined.
If the at least one CpG within the region of SEQ ID NO: 23 is methylated, said methylation is indicative of the presence of an adenoma of the colon.

In one embodiment the methylation status of the CpG rich region of SEQ ID NO: 25 is determined by evaluating the methylation status of at least one CpG within the region of SEQ ID NO: 25, more preferably within the region +/- 200 base pairs from position 126 and/or within the region +/- 200 base pairs from position 258 and/or within the region +/- 200 base pairs from position 447 and/or within the region +/- 200 base pairs from position 716 and/or within the region +/- 200 base pairs from position 868 and/or within the region +/- 200 base pairs from position 1060 in SEQ ID NO: 25, even more preferably within the region +/- 100 base pairs from position 126 and/or within the region +/- 100 base pairs from position 258 and/or within the region +/- 100 base pairs from position 447 and/or within the region +/- 100 base pairs from position 716 and/or within the region +/- 100 base pairs from position 868 and/or within the region +/- 100 base pairs from position 1060 in SEQ ID NO: 25, even more preferably within the region +/- 50 base pairs from position 126 and/or within the region +/- 50 base pairs from position 258 and/or within the region +/- 50 base pairs from position 447 and/or within the region +/- 50 base pairs from position 716 and/or within the region +/- 50 base pairs from position 868 and/or within the region +/- 50 base pairs from position 1060 in SEQ ID NO: 25 for the presence of methylated CpGs. In a most preferred embodiment the methylation status of the CpG of position 126 and/or position 258 and/or position 447 and/or position 716 and/or position 868 and/or position 1060 of SEQ ID NO: 25 is determined.
If the at least one CpG within the region of SEQ ID NO 25 is methylated, said methylation is indicative of the presence of an adenoma of the colon.

In one embodiment the methylation status of the CpG rich region of SEQ ID NO: 32 is determined by evaluating the methylation status of at least one CpG within the region of SEQ ID NO: 32, more preferably within the region +/- 200 base pairs from position 395 and/or within the region +/- 200 base pairs from position 605 and/or within the region +/- 200 base pairs from position 649 and/or within the region +/- 200 base pairs from position 693 and/or within the region +/- 200 base pairs from position 2922 in SEQ ID NO: 32, even more preferably within the region +/- 100 base pairs from position 395 and/or within the region +/- 100 base pairs from position 605 and/or within the region +/- 100 base pairs from position 649 and/or within the region +/- 100 base pairs from position 693 and/or within the region +/- 100 base pairs from position 2922 in SEQ ID NO: 32, even more preferably within the region +/- 50 base pairs from position 395 and/or within the region +/- 50 base pairs from position 605 and/or within the region +/-50 base pairs from position 649 and/or within the region +/- 50 base pairs from position 693 and/or within the region +/- 50 base pairs from position 2922 in SEQ ID NO: 32 for the presence of methylated CpGs. In a most preferred embodiment the methylation status of the CpG of position 395 and/or position 605 and/or position 649 and/or position 693 and/or position 2922 of SEQ ID NO: 32 is determined.
If the at least one CpG within the region of SEQ ID NO 32 is methylated, said methylation is indicative of the presence of an adenoma of the colon.

In one embodiment the methylation status of the CpG rich region of SEQ ID NO: 33 is determined by evaluating the methylation status of at least one CpG within the region of SEQ ID NO: 33, more preferably within the region +/- 200 base pairs from position 1240 and/or within the region +/- 200 base pairs from position 1450 and/or within the region +/- 200 base pairs from position 1461 and/or within the region +/- 200 base pairs from position 1683 in SEQ ID NO: 33, even more preferably within the region +/- 100 base pairs from position 1240 and/or within the region +/- 100 base pairs from position 1450 and/or within the region +/- 100 base pairs from position 1461 and/or within the region +/- 100 base pairs from position 1683 in SEQ ID NO: 33, even more preferably within the region +/- 50 base pairs from position 1240 and/or within the region +/- 50 base pairs from position 1450 and/or within the region +/- 50 base pairs from position 1461 and/or within the region +/- 50 base pairs from position 1683 in SEQ ID NO: 33 for the presence of methylated CpGs. In a most preferred embodiment the methylation status of the CpG of position 1240 and/or position 1450 and/or position 1461 and/or position 1683 of SEQ ID NO: 33 is determined.
If the at least one CpG within the region of SEQ ID NO 33 is methylated, said methylation is indicative of the presence of an adenoma of the colon.

In one embodiment the methylation status of the CpG rich region of SEQ ID NO: 35 is determined by evaluating the methylation status of at least one CpG within the region of SEQ ID NO: 35, more preferably within the region +/- 200 base pairs from position 1484 and/or within the region +/-200 base pairs from position 1875 in SEQ ID NO: 35, even more preferably within the region +/- 100 base pairs from position 1484 and/or within the region +/- 100 base pairs from position 1875 in SEQ ID NO: 35, even more preferably within the region +/- 50 base pairs from position 1484 and/or within the region +/- 50 base pairs from position 1875 in SEQ ID NO: 35 for the presence of methylated CpGs. In a most preferred embodiment the methylation status of the CpG of position 1484 and/or position 1875 of SEQ ID NO: 35 is determined.
If the at least one CpG within the region of SEQ ID NO 35 is methylated, said methylation is indicative of the presence of an adenoma of the colon.

In one embodiment the methylation status of the CpG rich region of SEQ ID NO: 36 is determined by evaluating the methylation status of at least one CpG within the region of SEQ ID NO: 36, more preferably within the region +/- 200 base pairs from position 1406 and/or within the region +/- 200 base pairs from position 1586 and/or within the region +/- 200 base pairs from position 1621 and/or within the region +/- 200 base pairs from position 2051 in SEQ ID NO: 36, even more preferably within the region +/- 100 base pairs from position 1406 and/or within the region +/- 100 base pairs from position 1586 and/or within the region +/- 100 base pairs from position 1621 and/or within the region +/- 100 base pairs from position 2051 in SEQ ID NO: 36, even more preferably within the region +/- 50 base pairs from position 1406 and/or within the region +/- 50 base pairs from position 1586 and/or within the region +/- 50 base pairs from position 1621 and/or within the region +/- 50 base pairs from position 2051 in SEQ ID NO: 36 for the presence of methylated CpGs. In a most preferred embodiment the methylation status of the CpG of position 1406 and/or position 1586 and/or position 1621 and/or position 2051 of SEQ ID NO: 36 is determined.
If the at least one CpG within the region of SEQ ID NO 36 is methylated, said methylation is indicative of the presence of an adenoma of the colon.

In one embodiment the methylation status of the CpG rich region of SEQ ID NO: 38 is determined by evaluating the methylation status of at least one CpG within the region of SEQ ID NO: 38, more preferably within the region +/- 200 base pairs from position 130 and/or within the region +/-200 base pairs from position 213 and/or within the region +/- 200 base pairs from position 220 and/or within the region +/- 200 base pairs from position 399 and/or within the region +/- 200 base pairs from position 419 and/or within the region +/- 200 base pairs from position 897 and/or within the region +/- 200 base pairs from position 1348 in SEQ ID NO: 38, even more preferably within the region +/- 100 base pairs from position 130 and/or within the region +/- 100 base pairs from position 213 and/or within the region +/- 100 base pairs from position 220 and/or within the region +/- 100 base pairs from position 399 and/or within the region +/- 100 base pairs from position 419 and/or within the region +/- 100 base pairs from position 897 and/or within the region +/- 100 base pairs from position 1348 in SEQ ID NO: 38, even more preferably within the region +/- 50 base pairs from position 130 and/or within the region +/- 50 base pairs from position 213 and/or within the region +/- 50 base pairs from position 220 and/or within the region +/- 50 base pairs from position 399 and/or within the region +/- 50 base pairs from position 419 and/or within the region +/- 50 base pairs from position 897 and/or within the region +/- 50 base pairs from position 1348 in SEQ ID NO: 38 for the presence of methylated CpGs. In a most preferred embodiment the methylation status of the CpG of position 130 and/or position 213 and/or position 220 and/or position 399 and/or position 419 and/or position 897 and/or position 1348 of SEQ ID NO: 38 is determined.
If the at least one CpG within the region of SEQ ID NO 38 is methylated, said methylation is indicative of the presence of an adenoma of the colon.

In one embodiment the methylation status of the CpG rich region of SEQ ID NO: 44 is determined by evaluating the methylation status of at least one CpG within the region of SEQ ID NO: 44, more preferably within the region +/- 200 base pairs from position 315 and/or within the region +/-200 base pairs from position 317 and/or within the region +/- 200 base pairs from position 327 and/or within the region +/- 200 base pairs from position 349 in SEQ ID NO: 44, even more preferably within the region +/- 100 base pairs from position 315 and/or within the region +/- 100 base pairs from position 317 and/or within the region +/- 100 base pairs from position 327 and/or within the region +/- 100 base pairs from position 349 in SEQ ID NO: 44, even more preferably within the region +/- 50 base pairs from position 315 and/or within the region +/- 50 base pairs from position 317 and/or within the region +/- 50 base pairs from position 327 and/or within the region +/- 50 base pairs from position 349 in SEQ ID NO: 44 for the presence of methylated CpGs. In a most preferred embodiment the methylation status of the CpG of position 315 and/or position 317 and/or position 327 and/or position 349 of SEQ ID NO: 44 is determined.
If the at least one CpG within the region of SEQ ID NO 44 is methylated, said methylation is indicative of the presence of an adenoma of the colon.

In one embodiment the methylation status of the CpG rich region of SEQ ID NO: 46 is determined by evaluating the methylation status of at least one CpG within the region of SEQ ID NO: 46, more preferably within the region +/- 150 base pairs from position 200 in SEQ ID NO: 46, even more preferably within the region +/- 100 base pairs from position 200 in SEQ ID NO: 46, even more preferably within the region +/- 50 base pairs from position 200 in SEQ ID NO: 46 for the presence of methylated CpGs. In a most preferred embodiment the methylation status of the CpG of position 200 of SEQ ID NO: 46 is determined.
If the at least one CpG within the region of SEQ ID NO 46 is methylated, said methylation is indicative of the presence of an adenoma of the colon.

In one embodiment the methylation status of the CpG rich region of SEQ ID NO: 46 is determined by evaluating the methylation status of at least one CpG within the region of SEQ ID NO: 46, more preferably within the region +/- 150 base pairs from position 200 in SEQ ID NO: 46, even more preferably within the region +/- 100 base pairs from position 200 in SEQ ID NO: 46, even more preferably within the region +/- 50 base pairs from position 200 in SEQ ID NO: 46 for the presence of methylated CpGs. In a most preferred embodiment the methylation status of the CpG of position 200 of SEQ ID NO: 46 is determined.
If the at least one CpG within the region of SEQ ID NO 46 is methylated, said methylation is indicative of the presence of an adenoma of the colon.

In one embodiment the methylation status of the CpG rich region of SEQ ID NO: 47 is determined by evaluating the methylation status of at least one CpG within the region of SEQ ID NO: 47, more preferably within the region +/- 150 base pairs from position 200 in SEQ ID NO: 47, even more preferably within the region +/- 100 base pairs from position 200 in SEQ ID NO: 47, even more preferably within the region +/- 50 base pairs from position 200 in SEQ ID NO: 47 for the presence of methylated CpGs. In a most preferred embodiment the methylation status of the CpG of position 200 of SEQ ID NO: 47 is determined.
If the at least one CpG within the region of SEQ ID NO 47 is methylated, said methylation is indicative of the presence of an adenoma of the colon.

In one embodiment the methylation status of the CpG rich region of SEQ ID NO: 48 is determined by evaluating the methylation status of at least one CpG within the region of SEQ ID NO: 48, more preferably within the region +/- 150 base pairs from position 200 in SEQ ID NO: 48, even more preferably within the region +/- 100 base pairs from position 200 in SEQ ID NO: 48, even more preferably within the region +/- 50 base pairs from position 200 in SEQ ID NO: 48 for the presence of methylated CpGs. In a most preferred embodiment the methylation status of the CpG of position 200 of SEQ ID NO: 48 is determined.
If the at least one CpG within the region of SEQ ID NO 48 is methylated, said methylation is indicative of the presence of an adenoma of the colon.

In one embodiment the methylation status of the CpG rich region of SEQ ID NO: 49 is determined by evaluating the methylation status of at least one CpG within the region of SEQ ID NO: 49, more preferably within the region +/- 150 base pairs from position 200 in SEQ ID NO: 49, even more preferably within the region +/- 100 base pairs from position 200 in SEQ ID NO: 49, even more preferably within the region +/- 50 base pairs from position 200 in SEQ ID NO: 49 for the presence of methylated CpGs. In a most preferred embodiment the methylation status of the CpG of position 200 of SEQ ID NO: 49 is determined.
If the at least one CpG within the region of SEQ ID NO 49 is methylated, said methylation is indicative of the presence of an adenoma of the colon.

In one embodiment the methylation status of the CpG rich region of SEQ ID NO: 50 is determined by evaluating the methylation status of at least one CpG within the region of SEQ ID NO: 50, more preferably within the region +/- 150 base pairs from position 200 in SEQ ID NO: 50, even more preferably within the region +/- 100 base pairs from position 200 in SEQ ID NO: 50, even more preferably within the region +/- 50 base pairs from position 200 in SEQ ID NO: 50 for the presence of methylated CpGs. In a most preferred embodiment the methylation status of the CpG of position 200 of SEQ ID NO: 50 is determined.
If the at least one CpG within the region of SEQ ID NO 50 is methylated, said methylation is indicative of the presence of an adenoma of the colon.

In one embodiment the methylation status of the CpG rich region of SEQ ID NO: 51 is determined by evaluating the methylation status of at least one CpG within the region of SEQ ID NO: 51, more preferably within the region +/- 150 base pairs from position 200 in SEQ ID NO: 51, even more preferably within the region +/- 100 base pairs from position 200 in SEQ ID NO: 51, even more preferably within the region +/- 50 base pairs from position 200 in SEQ ID NO: 51 for the presence of methylated CpGs. In a most preferred embodiment the methylation status of the CpG of position 200 of SEQ ID NO: 51 is determined.
If the at least one CpG within the region of SEQ ID NO 51 is methylated, said methylation is indicative of the presence of an adenoma of the colon.

In one embodiment the methylation status of the CpG rich region of SEQ ID NO: 52 is determined by evaluating the methylation status of at least one CpG within the region of SEQ ID NO: 52, more preferably within the region +/- 150 base pairs from position 200 in SEQ ID NO: 52, even more preferably within the region +/- 100 base pairs from position 200 in SEQ ID NO: 52, even more preferably within the region +/- 50 base pairs from position 200 in SEQ ID NO: 52 for the presence of methylated CpGs. In a most preferred embodiment the methylation status of the CpG of position 200 of SEQ ID NO: 52 is determined.
If the at least one CpG within the region of SEQ ID NO 52 is methylated, said methylation is indicative of the presence of an adenoma of the colon.

In one embodiment the methylation status of the CpG rich region of SEQ ID NO: 53 is determined by evaluating the methylation status of at least one CpG within the region of SEQ ID NO: 53, more preferably within the region +/- 150 base pairs from position 200 in SEQ ID NO: 53, even more preferably within the region +/- 100 base pairs from position 200 in SEQ ID NO: 53, even more preferably within the region +/- 50 base pairs from position 200 in SEQ ID NO: 53 for the presence of methylated CpGs. In a most preferred embodiment the methylation status of the CpG of position 200 of SEQ ID NO: 53 is determined.
If the at least one CpG within the region of SEQ ID NO 53 is methylated, said methylation is indicative of the presence of an adenoma of the colon.

In one embodiment the methylation status of the CpG rich region of SEQ ID NO: 54 is determined by evaluating the methylation status of at least one CpG within the region of SEQ ID NO: 54, more preferably within the region +/- 150 base pairs from position 200 in SEQ ID NO: 54, even more preferably within the region +/- 100 base pairs from position 200 in SEQ ID NO: 54, even more preferably within the region +/- 50 base pairs from position 200 in SEQ ID NO: 54 for the presence of methylated CpGs. In a most preferred embodiment the methylation status of the CpG of position 200 of SEQ ID NO: 54 is determined.
If the at least one CpG within the region of SEQ ID NO 54 is methylated, said methylation is indicative of the presence of an adenoma of the colon.

In one embodiment the methylation status of the CpG rich region of SEQ ID NO: 55 is determined by evaluating the methylation status of at least one CpG within the region of SEQ ID NO: 55, more preferably within the region +/- 150 base pairs from position 200 in SEQ ID NO: 55, even more preferably within the region +/- 100 base pairs from position 200 in SEQ ID NO: 55, even more preferably within the region +/- 50 base pairs from position 200 in SEQ ID NO: 55 for the presence of methylated CpGs. In a most preferred embodiment the methylation status of the CpG of position 200 of SEQ ID NO: 55 is determined.
If the at least one CpG within the region of SEQ ID NO 55 is methylated, said methylation is indicative of the presence of an adenoma of the colon.

In one embodiment the methylation status of the CpG rich region of SEQ ID NO: 56 is determined by evaluating the methylation status of at least one CpG within the region of SEQ ID NO: 56, more preferably within the region +/- 150 base pairs from position 200 in SEQ ID NO: 56, even more preferably within the region +/- 100 base pairs from position 200 in SEQ ID NO: 56, even more preferably within the region +/- 50 base pairs from position 200 in SEQ ID NO: 56 for the presence of methylated CpGs. In a most preferred embodiment the methylation status of the CpG of position 200 of SEQ ID NO: 56 is determined.
If the at least one CpG within the region of SEQ ID NO 56 is methylated, said methylation is indicative of the presence of an adenoma of the colon.

In one embodiment the methylation status of the CpG rich region of SEQ ID NO: 57 is determined by evaluating the methylation status of at least one CpG within the region of SEQ ID NO: 57, more preferably within the region +/- 150 base pairs from position 200 in SEQ ID NO: 57, even more preferably within the region +/- 100 base pairs from position 200 in SEQ ID NO: 57, even more preferably within the region +/- 50 base pairs from position 200 in SEQ ID NO: 57 for the presence of methylated CpGs. In a most preferred embodiment the methylation status of the CpG of position 200 of SEQ ID NO: 57 is determined.

If the at least one CpG within the region of SEQ ID NO 57 is methylated, said methylation is indicative of the presence of an adenoma of the colon.

In one embodiment the methylation status of the CpG rich region of SEQ ID NO: 58 is determined by evaluating the methylation status of at least one CpG within the region of SEQ ID NO: 58, more preferably within the region +/- 150 base pairs from position 200 in SEQ ID NO: 58, even more preferably within the region +/- 100 base pairs from position 200 in SEQ ID NO: 58, even more preferably within the region +/- 50 base pairs from position 200 in SEQ ID NO: 58 for the presence of methylated CpGs. In a most preferred embodiment the methylation status of the CpG of position 200 of SEQ ID NO: 58 is determined.
If the at least one CpG within the region of SEQ ID NO 58 is methylated, said methylation is indicative of the presence of an adenoma of the colon.

In one embodiment the methylation status of the CpG rich region of SEQ ID NO: 59 is determined by evaluating the methylation status of at least one CpG within the region of SEQ ID NO: 59, more preferably within the region +/- 150 base pairs from position 200 in SEQ ID NO: 59, even more preferably within the region +/- 100 base pairs from position 200 in SEQ ID NO: 59, even more preferably within the region +/- 50 base pairs from position 200 in SEQ ID NO: 59 for the presence of methylated CpGs. In a most preferred embodiment the methylation status of the CpG of position 200 of SEQ ID NO: 59 is determined.
If the at least one CpG within the region of SEQ ID NO 59 is methylated, said methylation is indicative of the presence of an adenoma of the colon.

In one embodiment the methylation status of the CpG rich region of SEQ ID NO: 60 is determined by evaluating the methylation status of at least one CpG within the region of SEQ ID NO: 60, more preferably within the region +/- 150 base pairs from position 200 in SEQ ID NO: 60, even more preferably within the region +/- 100 base pairs from position 200 in SEQ ID NO: 60, even more preferably within the region +/- 50 base pairs from position 200 in SEQ ID NO: 60 for the presence of methylated CpGs. In a most preferred embodiment the methylation status of the CpG of position 200 of SEQ ID NO: 60 is determined.
If the at least one CpG within the region of SEQ ID NO 60 is methylated, said methylation is indicative of the presence of an adenoma of the colon.

In one embodiment the methylation status of the CpG rich region of SEQ ID NO: 61 is determined by evaluating the methylation status of at least one CpG within the region of SEQ ID NO: 61, more preferably within the region +/- 150 base pairs from position 200 in SEQ ID NO: 61, even more preferably within the region +/- 100 base pairs from position 200 in SEQ ID NO: 61, even more preferably within the region +/- 50 base pairs from position 200 in SEQ ID NO: 61 for the presence of methylated CpGs. In a most preferred embodiment the methylation status of the CpG of position 200 of SEQ ID NO: 61 is determined.
If the at least one CpG within the region of SEQ ID NO 61 is methylated, said methylation is indicative of the presence of an adenoma of the colon.

In one embodiment the methylation status of the CpG rich region of SEQ ID NO: 62 is determined by evaluating the methylation status of at least one CpG within the region of SEQ ID NO: 62, more preferably within the region +/- 150 base pairs from position 200 in SEQ ID NO: 62, even more preferably within the region +/- 100 base pairs from position 200 in SEQ ID NO: 62, even more preferably within the region +/- 50 base pairs from position 200 in SEQ ID NO: 62 for the presence of methylated CpGs. In a most preferred embodiment the methylation status of the CpG of position 200 of SEQ ID NO: 62 is determined.
If the at least one CpG within the region of SEQ ID NO 62 is methylated, said methylation is indicative of the presence of an adenoma of the colon.

In one embodiment the methylation status of the CpG rich region of SEQ ID NO: 63 is determined by evaluating the methylation status of at least one CpG within the region of SEQ ID NO: 63, more preferably within the region +/- 150 base pairs from position 200 in SEQ ID NO: 63, even more preferably within the region +/- 100 base pairs from position 200 in SEQ ID NO: 63, even more preferably within the region +/- 50 base pairs from position 200 in SEQ ID NO: 63 for the presence of methylated CpGs. In a most preferred embodiment the methylation status of the CpG of position 200 of SEQ ID NO: 63 is determined.
If the at least one CpG within the region of SEQ ID NO 63 is methylated, said methylation is indicative of the presence of an adenoma of the colon.

In one embodiment the methylation status of the CpG rich region of SEQ ID NO: 64 is determined by evaluating the methylation status of at least one CpG within the region of SEQ ID NO: 64, more preferably within the region +/- 150 base pairs from position 200 in SEQ ID NO: 64, even more preferably within the region +/- 100 base pairs from position 200 in SEQ ID NO: 64, even more preferably within the region +/- 50 base pairs from position 200 in SEQ ID NO: 64 for the presence of methylated CpGs. In a most preferred embodiment the methylation status of the CpG of position 200 of SEQ ID NO: 64 is determined.
If the at least one CpG within the region of SEQ ID NO 64 is methylated, said methylation is indicative of the presence of an adenoma of the colon.

In one embodiment the methylation status of the CpG rich region of SEQ ID NO: 65 is determined by evaluating the methylation status of at least one CpG within the region of SEQ ID NO: 65, more preferably within the region +/- 150 base pairs from position 200 in SEQ ID NO: 65, even more preferably within the region +/- 100 base pairs from position 200 in SEQ ID NO: 65, even more preferably within the region +/- 50 base pairs from position 200 in SEQ ID NO: 65 for the presence of methylated CpGs. In a most preferred embodiment the methylation status of the CpG of position 200 of SEQ ID NO: 65 is determined.
If the at least one CpG within the region of SEQ ID NO 65 is methylated, said methylation is indicative of the presence of an adenoma of the colon.

In one embodiment the methylation status of the CpG rich region of SEQ ID NO: 66 is determined by evaluating the methylation status of at least one CpG within the region of SEQ ID NO: 66, more preferably within the region +/- 150 base pairs from position 200 in SEQ ID NO: 66, even more preferably within the region +/- 100 base pairs from position 200 in SEQ ID NO: 66, even more preferably within the region +/- 50 base pairs from position 200 in SEQ ID NO: 66 for the presence of methylated CpGs. In a most preferred embodiment the methylation status of the CpG of position 200 of SEQ ID NO: 66 is determined.
If the at least one CpG within the region of SEQ ID NO 66 is methylated, said methylation is indicative of the presence of an adenoma of the colon.

In one embodiment the methylation status of the CpG rich region of SEQ ID NO: 67 is determined by evaluating the methylation status of at least one CpG within the region of SEQ ID NO: 67, more preferably within the region +/- 150 base pairs from position 200 in SEQ ID NO: 67, even more preferably within the region +/- 100 base pairs from position 200 in SEQ ID NO: 67, even more preferably within the region +/- 50 base pairs from position 200 in SEQ ID NO: 67 for the presence of methylated CpGs. In a most preferred embodiment the methylation status of the CpG of position 200 of SEQ ID NO: 67 is determined.
If the at least one CpG within the region of SEQ ID NO 67 is methylated, said methylation is indicative of the presence of an adenoma of the colon.

In one embodiment the methylation status of the CpG rich region of SEQ ID NO: 68 is determined by evaluating the methylation status of at least one CpG within the region of SEQ ID NO: 68, more preferably within the region +/- 150 base pairs from position 200 in SEQ ID NO: 68, even more preferably within the region +/- 100 base pairs from position 200 in SEQ ID NO: 68, even more preferably within the region +/- 50 base pairs from position 200 in SEQ ID NO: 68 for the presence of methylated CpGs. In a most preferred embodiment the methylation status of the CpG of position 200 of SEQ ID NO: 68 is determined.
If the at least one CpG within the region of SEQ ID NO 68 is methylated, said methylation is indicative of the presence of an adenoma of the colon.

In one embodiment the methylation status of the CpG rich region of SEQ ID NO: 69 is determined by evaluating the methylation status of at least one CpG within the region of SEQ ID NO: 69, more preferably within the region +/- 150 base pairs from position 200 in SEQ ID NO: 69, even more preferably within the region +/- 100 base pairs from position 200 in SEQ ID NO: 69, even more preferably within the region +/- 50 base pairs from position 200 in SEQ ID NO: 69 for the presence of methylated CpGs. In a most preferred embodiment the methylation status of the CpG of position 200 of SEQ ID NO: 69 is determined.
If the at least one CpG within the region of SEQ ID NO 69 is methylated, said methylation is indicative of the presence of an adenoma of the colon.

In one embodiment the methylation status of the CpG rich region of SEQ ID NO: 70 is determined by evaluating the methylation status of at least one CpG within the region of SEQ ID NO: 70, more preferably within the region +/- 150 base pairs from position 200 in SEQ ID NO: 70, even more preferably within the region +/- 100 base pairs from position 200 in SEQ ID NO: 70, even more preferably within the region +/- 50 base pairs from position 200 in SEQ ID NO: 70 for the presence of methylated CpGs. In a most preferred embodiment the methylation status of the CpG of position 200 of SEQ ID NO: 70 is determined.
If the at least one CpG within the region of SEQ ID NO 70 is methylated, said methylation is indicative of the presence of an adenoma of the colon.

In one embodiment the methylation status of the CpG rich region of SEQ ID NO: 71 is determined by evaluating the methylation status of at least one CpG within the region of SEQ ID NO: 71, more preferably within the region +/- 150 base pairs from position 200 in SEQ ID NO: 71, even more preferably within the region +/- 100 base pairs from position 200 in SEQ ID NO: 71, even more preferably within the region +/- 50 base pairs from position 200 in SEQ ID NO: 71 for the presence of methylated CpGs. In a most preferred embodiment the methylation status of the CpG of position 200 of SEQ ID NO: 71 is determined.
If the at least one CpG within the region of SEQ ID NO 71 is methylated, said methylation is indicative of the presence of an adenoma of the colon.

In one embodiment the methylation status of the CpG rich region of SEQ ID NO: 72 is determined by evaluating the methylation status of at least one CpG within the region of SEQ ID NO: 72, more preferably within the region +/- 150 base pairs from position 200 in SEQ ID NO: 72, even more preferably within the region +/- 100 base pairs from position 200 in SEQ ID NO: 72, even more preferably within the region +/- 50 base pairs from position 200 in SEQ ID NO: 72 for the presence of methylated CpGs. In a most preferred embodiment the methylation status of the CpG of position 200 of SEQ ID NO: 72 is determined.
If the at least one CpG within the region of SEQ ID NO 72 is methylated, said methylation is indicative of the presence of an adenoma of the colon.

In one embodiment the methylation status of the CpG rich region of SEQ ID NO: 73 is determined by evaluating the methylation status of at least one CpG within the region of SEQ ID NO: 73, more preferably within the region +/- 150 base pairs from position 200 in SEQ ID NO: 73, even more preferably within the region +/- 100 base pairs from position 200 in SEQ ID NO: 73, even more preferably within the region +/- 50 base pairs from position 200 in SEQ ID NO: 73 for the presence of methylated CpGs. In a most preferred embodiment the methylation status of the CpG of position 200 of SEQ ID NO: 73 is determined.
If the at least one CpG within the region of SEQ ID NO 73 is methylated, said methylation is indicative of the presence of an adenoma of the colon.

In one embodiment the methylation status of the CpG rich region of SEQ ID NO: 74 is determined by evaluating the methylation status of at least one CpG within the region of SEQ ID NO: 74, more preferably within the region +/- 150 base pairs from position 200 in SEQ ID NO: 74, even more preferably within the region +/- 100 base pairs from position 200 in SEQ ID NO: 74, even more preferably within the region +/- 50 base pairs from position 200 in SEQ ID NO: 74 for the presence of methylated CpGs. In a most preferred embodiment the methylation status of the CpG of position 200 of SEQ ID NO: 74 is determined.
If the at least one CpG within the region of SEQ ID NO 74 is methylated, said methylation is indicative of the presence of an adenoma of the colon.

In one embodiment the methylation status of the CpG rich region of SEQ ID NO: 75 is determined by evaluating the methylation status of at least one CpG within the region of SEQ ID NO: 75, more preferably within the region +/- 150 base pairs from position 200 in SEQ ID NO: 75, even more preferably within the region +/- 100 base pairs from position 200 in SEQ ID NO: 75, even more preferably within the region +/- 50 base pairs from position 200 in SEQ ID NO: 75 for the presence of methylated CpGs. In a most preferred embodiment the methylation status of the CpG of position 200 of SEQ ID NO: 75 is determined.
If the at least one CpG within the region of SEQ ID NO 75 is methylated, said methylation is indicative of the presence of an adenoma of the colon.

In one embodiment the methylation status of the CpG rich region of SEQ ID NO: 76 is determined by evaluating the methylation status of at least one CpG within the region of SEQ ID NO: 76, more preferably within the region +/- 150 base pairs from position 200 in SEQ ID NO: 76, even more preferably within the region +/- 100 base pairs from position 200 in SEQ ID NO: 76, even more preferably within the region +/- 50 base pairs from position 200 in SEQ ID NO: 76 for the presence of methylated CpGs. In a most preferred embodiment the methylation status of the CpG of position 200 of SEQ ID NO: 76 is determined.

If the at least one CpG within the region of SEQ ID NO 76 is methylated, said methylation is indicative of the presence of an adenoma of the colon.

In one embodiment the methylation status of the CpG rich region of SEQ ID NO: 77 is determined by evaluating the methylation status of at least one CpG within the region of SEQ ID NO: 77, more preferably within the region +/- 150 base pairs from position 200 in SEQ ID NO: 77, even more preferably within the region +/- 100 base pairs from position 200 in SEQ ID NO: 77, even more preferably within the region +/- 50 base pairs from position 200 in SEQ ID NO: 77 for the presence of methylated CpGs. In a most preferred embodiment the methylation status of the CpG of position 200 of SEQ ID NO: 77 is determined.
If the at least one CpG within the region of SEQ ID NO 77 is methylated, said methylation is indicative of the presence of an adenoma of the colon.

In one embodiment the methylation status of the CpG rich region of SEQ ID NO: 78 is determined by evaluating the methylation status of at least one CpG within the region of SEQ ID NO: 78, more preferably within the region +/- 150 base pairs from position 200 in SEQ ID NO: 78, even more preferably within the region +/- 100 base pairs from position 200 in SEQ ID NO: 78, even more preferably within the region +/- 50 base pairs from position 200 in SEQ ID NO: 78 for the presence of methylated CpGs. In a most preferred embodiment the methylation status of the CpG of position 200 of SEQ ID NO: 78 is determined.
If the at least one CpG within the region of SEQ ID NO 78 is methylated, said methylation is indicative of the presence of an adenoma of the colon.

In one embodiment the methylation status of the CpG rich region of SEQ ID NO: 79 is determined by evaluating the methylation status of at least one CpG within the region of SEQ ID NO: 79, more preferably within the region +/- 150 base pairs from position 200 in SEQ ID NO: 79, even more preferably within the region +/- 100 base pairs from position 200 in SEQ ID NO: 79, even more preferably within the region +/- 50 base pairs from position 200 in SEQ ID NO: 79 for the presence of methylated CpGs. In a most preferred embodiment the methylation status of the CpG of position 200 of SEQ ID NO: 79 is determined.
If the at least one CpG within the region of SEQ ID NO 79 is methylated, said methylation is indicative of the presence of an adenoma of the colon.

In one embodiment the methylation status of the CpG rich region of SEQ ID NO: 80 is determined by evaluating the methylation status of at least one CpG within the region of SEQ ID NO: 80, more preferably within the region +/- 150 base pairs from position 200 in SEQ ID NO: 80, even more preferably within the region +/- 100 base pairs from position 200 in SEQ ID NO: 80, even more preferably within the region +/- 50 base pairs from position 200 in SEQ ID NO: 80 for the presence of methylated CpGs. In a most preferred embodiment the methylation status of the CpG of position 200 of SEQ ID NO: 80 is determined.
If the at least one CpG within the region of SEQ ID NO 80 is methylated, said methylation is indicative of the presence of an adenoma of the colon.

In one embodiment the methylation status of the CpG rich region of SEQ ID NO: 81 is determined by evaluating the methylation status of at least one CpG within the region of SEQ ID NO: 81, more preferably within the region +/- 150 base pairs from position 200 in SEQ ID NO: 81, even more preferably within the region +/- 100 base pairs from position 200 in SEQ ID NO: 81, even more preferably within the region +/- 50 base pairs from position 200 in SEQ ID NO: 81 for the presence of methylated CpGs. In a most preferred embodiment the methylation status of the CpG of position 200 of SEQ ID NO: 81 is determined.
If the at least one CpG within the region of SEQ ID NO 81 is methylated, said methylation is indicative of the presence of an adenoma of the colon.

In one embodiment the methylation status of the CpG rich region of SEQ ID NO: 82 is determined by evaluating the methylation status of at least one CpG within the region of SEQ ID NO: 82, more preferably within the region +/- 150 base pairs from position 200 in SEQ ID NO: 82, even more preferably within the region +/- 100 base pairs from position 200 in SEQ ID NO: 82, even more preferably within the region +/- 50 base pairs from position 200 in SEQ ID NO: 82 for the presence of methylated CpGs. In a most preferred embodiment the methylation status of the CpG of position 200 of SEQ ID NO: 82 is determined.
If the at least one CpG within the region of SEQ ID NO 82 is methylated, said methylation is indicative of the presence of an adenoma of the colon.

In one embodiment the methylation status of the CpG rich region of SEQ ID NO: 83 is determined by evaluating the methylation status of at least one CpG within the region of SEQ ID NO: 83, more preferably within the region +/- 150 base pairs from position 200 in SEQ ID NO: 83, even more preferably within the region +/- 100 base pairs from position 200 in SEQ ID NO: 83, even more preferably within the region +/- 50 base pairs from position 200 in SEQ ID NO: 83 for the presence of methylated CpGs. In a most preferred embodiment the methylation status of the CpG of position 200 of SEQ ID NO: 83 is determined.
If the at least one CpG within the region of SEQ ID NO 83 is methylated, said methylation is indicative of the presence of an adenoma of the colon.

In one embodiment the methylation status of the CpG rich region of SEQ ID NO: 84 is determined by evaluating the methylation status of at least one CpG within the region of SEQ ID NO: 84, more preferably within the region +/- 150 base pairs from position 200 in SEQ ID NO: 84, even more preferably within the region +/- 100 base pairs from position 200 in SEQ ID NO: 84, even more preferably within the region +/- 50 base pairs from position 200 in SEQ ID NO: 84 for the presence of methylated CpGs. In a most preferred embodiment the methylation status of the CpG of position 200 of SEQ ID NO: 84 is determined.
If the at least one CpG within the region of SEQ ID NO 84 is methylated, said methylation is indicative of the presence of an adenoma of the colon.

In one embodiment the methylation status of the CpG rich region of SEQ ID NO: 85 is determined by evaluating the methylation status of at least one CpG within the region of SEQ ID NO: 85, more preferably within the region +/- 150 base pairs from position 200 in SEQ ID NO: 85, even more preferably within the region +/- 100 base pairs from position 200 in SEQ ID NO: 85, even more preferably within the region +/- 50 base pairs from position 200 in SEQ ID NO: 85 for the presence of methylated CpGs. In a most preferred embodiment the methylation status of the CpG of position 200 of SEQ ID NO: 85 is determined.
If the at least one CpG within the region of SEQ ID NO 85 is methylated, said methylation is indicative of the presence of an adenoma of the colon.

In one embodiment the methylation status of the CpG rich region of SEQ ID NO: 86 is determined by evaluating the methylation status of at least one CpG within the region of SEQ ID NO: 86, more preferably within the region +/- 150 base pairs from position 200 in SEQ ID NO: 86, even more preferably within the region +/- 100 base pairs from position 200 in SEQ ID NO: 86, even more preferably within the region +/- 50 base pairs from position 200 in SEQ ID NO: 86 for the presence of methylated CpGs. In a most preferred embodiment the methylation status of the CpG of position 200 of SEQ ID NO: 86 is determined.
If the at least one CpG within the region of SEQ ID NO 86 is methylated, said methylation is indicative of the presence of an adenoma of the colon.

In one embodiment the methylation status of the CpG rich region of SEQ ID NO: 87 is determined by evaluating the methylation status of at least one CpG within the region of SEQ ID NO: 87, more preferably within the region +/- 150 base pairs from position 200 in SEQ ID NO: 87, even more preferably within the region +/- 100 base pairs from position 200 in SEQ ID NO: 87, even more preferably within the region +/- 50 base pairs from position 200 in SEQ ID NO: 87 for the presence of methylated CpGs. In a most preferred embodiment the methylation status of the CpG of position 200 of SEQ ID NO: 87 is determined.
If the at least one CpG within the region of SEQ ID NO. 87 is methylated, said methylation is indicative of the presence of an adenoma of the colon.

In one embodiment the methylation status of the CpG rich region of SEQ ID NO: 88 is determined by evaluating the methylation status of at least one CpG within the region of SEQ ID NO: 88, more preferably within the region +/- 150 base pairs from position 200 in SEQ ID NO: 88, even more preferably within the region +/- 100 base pairs from position 200 in SEQ ID NO: 88, even more preferably within the region +/- 50 base pairs from position 200 in SEQ ID NO: 88 for the presence of methylated CpGs. In a most preferred embodiment the methylation status of the CpG of position 200 of SEQ ID NO: 88 is determined.
If the at least one CpG within the region of SEQ ID NO 88 is methylated, said methylation is indicative of the presence of an adenoma of the colon.

In one embodiment the methylation status of the CpG rich region of SEQ ID NO: 89 is determined by evaluating the methylation status of at least one CpG within the region of SEQ ID NO: 89, more preferably within the region +/- 150 base pairs from position 200 in SEQ ID NO: 89, even more preferably within the region +/- 100 base pairs from position 200 in SEQ ID NO: 89, even more preferably within the region +/- 50 base pairs from position 200 in SEQ ID NO: 89 for the presence of methylated CpGs. In a most preferred embodiment the methylation status of the CpG of position 200 of SEQ ID NO: 89 is determined.
If the at least one CpG within the region of SEQ ID NO 89 is methylated, said methylation is indicative of the presence of an adenoma of the colon.

In one embodiment the methylation status of the CpG rich region of SEQ ID NO: 90 is determined by evaluating the methylation status of at least one CpG within the region of SEQ ID NO: 90, more preferably within the region +/- 150 base pairs from position 200 in SEQ ID NO: 90, even more preferably within the region +/- 100 base pairs from position 200 in SEQ ID NO: 90, even more preferably within the region +/- 50 base pairs from position 200 in SEQ ID NO: 90 for the presence of methylated CpGs. In a most preferred embodiment the methylation status of the CpG of position 200 of SEQ ID NO: 90 is determined.
If the at least one CpG within the region of SEQ ID NO: 90 is methylated, said methylation is indicative of the presence of an adenoma of the colon.

In one embodiment the methylation status of the CpG rich region of SEQ ID NO: 91 is determined by evaluating the methylation status of at least one CpG within the region of SEQ ID NO: 91, more preferably within the region +/- 150 base pairs from position 200 in SEQ ID NO: 91, even more preferably within the region +/- 100 base pairs from position 200 in SEQ ID NO: 91, even more preferably within the region +/- 50 base pairs from position 200 in SEQ ID NO: 91 for the presence of methylated CpGs. In a most preferred embodiment the methylation status of the CpG of position 200 of SEQ ID NO: 91 is determined.
If the at least one CpG within the region of SEQ ID NO 91 is methylated, said methylation is indicative of the presence of an adenoma of the colon.

In one embodiment, the determination of the methylation status of the CpG at position 172 and/or 284 of SEQ ID NO: 1 is combined with the determination of the methylation status of at least one CpG present in the CpG rich region selected from the group consisting of: SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 226 and/or 831 of SEQ ID NO: 8 is combined with the determination of the methylation status of at least one CpG present in the CpG rich region selected from the group consisting of: SEQ ID NO: 1, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 101 and/or 643 of SEQ ID NO: 10 is combined with the determination of the methylation status of at least one CpG present in the CpG rich region selected from the group consisting of: SEQ ID NO: 1, SEQ ID NO: 8, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 612 and/or 668 and/or 835 of SEQ ID NO: 11 is combined with the determination of the methylation status of at least one CpG present in the CpG rich region selected from the group consisting of: SEQ ID NO: 1, SEQ ID NO: 8, SEQ ID NO: 10, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 231 and/or 1072 and/or 1074 and/or 1086 and/or 1097 and/or 1110 and/or 1115 and/or 1176 of SEQ ID NO: 18 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 292 and/or 603 and/or 612 and/or 694 and/or 1051 and/or 1330 of SEQ ID NO: 22 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 393 and/or 395 and/or 398 and/or 482 of SEQ ID NO: 23 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 126 and/or 258 and/or 447 and/or 716 and/or 868 and/or 1060 and/of SEQ ID NO: 25 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 395 and/or 605 and/or 649 and/or 693 and/or 2922 of SEQ ID NO: 32 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 1240 and/or 1450 and/or 1461 and/or 1683 of SEQ ID NO: 33 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 1484 and/or 1875 of SEQ ID NO: 35 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 1406 and/or 1586 and/or 1621 and/or 2051 of SEQ ID NO: 36 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 130 and/or 213 and/or 220 and/or 399 and/or 419 and/or 897 and/or 1348 of SEQ ID NO: 38 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 315 and/or 317 and/or 327 and/or 349 of SEQ ID NO: 44 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 46 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 47 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 48 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 49 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 50 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 51 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 52 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 53 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 54 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 55 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 56 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 57 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 58 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 59 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 60 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 61 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 62 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 63 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 64 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 65 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 66 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 67 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 68 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 69 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 70 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 71 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 72 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 73 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 74 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 75 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 76 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 77 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 78 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 79 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 80 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 81 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 82 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 83 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 84 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 85, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 85 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 86, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 86 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 87, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 87 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 88, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 88 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 89, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 89 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 90, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 90 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, and SEQ ID NO: 91. Preferably methylation of at least half, such as of at least two of said CpG is indicative of an adenoma of the colon.

In one embodiment, the determination of the methylation status of the CpG at position 200 of SEQ ID NO: 91 is combined with the determination of the methylation status of at least one CpG present in the CpG rich region selected from the group consisting of: SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, SEQ ID NO: 44, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, and SEQ ID NO: 90. Preferably methylation of at least half, such as of at least two of said CpG is indicative of an adenoma of the colon.

In one embodiment the methylation of a CpG rich region of the present invention is considered to be linked to the expression of a given gene. Accordingly methylation of said CpG rich region may influence the expression of said gene.

In one embodiment a CpG rich region according to the present disclosure is a CpG rich region which is linked to a gene selected from the group consisting of: HPSE2, NPY2R, PRICKLE1, FAM72B, FAM72A, ULBP1, ZNF568, PAX6, BARHL2, VCAN,
FAT4, ONECUT2, LRAT, HAND2, FAM72D, EBF2, FAM19A4, NALCN, CD40, MEOX2, ZNF804B, GRIA4, DLX5, SLC6A15, TBX5, SCAND3, FAM133A, NAP1L3, SSTR2, NKX2-2, PRMT8, ITGA4, DPP10, ADAMTS5, SNAP25, KCNA1, LRFN5, ZIC1, SEMA3D, TBCA, PCDH15, mir129-2 and ZNF606.

In another embodiment a CpG rich region according to the present disclosure is a CpG rich region which is linked to a gene selected from the group consisting of: : NPY2R, PRICKLE1, FAM72B, FAM72A, ULBP1, ZNF568, BARHL2, FAT4, FAM72D, EBF2, FAM19A4, NALCN, CD40, MEOX2, ZNF804B, DLX5, SCAND3, FAM133A, NKX2-2, PRMT8, DPP10, SNAP25, KCNA1, ZIC1, SEMA3D, TBCA, PCDH15, mir129-2 and ZNF606.

In another embodiement a CpG rich region according to the present disclosure is a CpG rich region which is linked to a gene selected from the group consisting of: ADAMTS5, DPP10, FAM19A4, HAND2, HPSE2, ITGA4, MIR129-2, PAX6, PRMT8, GRIA4, SLC6A15, TBX5, VCAN, ZNF568, ZNF804B, KCNA1, LRAT, DLX5, and BARHL2.

Without being bound by theory SEQ ID NO 1, SEQ ID NO 49 and SEQ ID NO 50 is considered linked to the sequence encoding HPSE2, SEQ ID NO 2 is considered linked to the sequence encoding NPY2R, SEQ ID NO 3 is linked to the sequence encoding PRICKLE1, SEQ ID NO 4 is considered linked to the sequence encoding FAM72B, SEQ ID NO 5 is considered linked to the sequence encoding FAM72A, SEQ ID NO 6 is considered linked to the sequence encoding ULBP1, SEQ ID NO 7, SEQ ID NO 70, SEQ ID NO 71, SEQ ID NO 72, SEQ ID NO 73, SEQ ID NO 74, SEQ ID NO 75, SEQ ID NO 76, SEQ ID NO 77, SEQ ID NO 78, SEQ ID NO 79, SEQ ID NO 80, SEQ ID NO 81, and SEQ ID NO 82 is considered linked to the sequence encoding ZNF568, SEQ ID NO 8, SEQ ID NO 53, SEQ ID NO 54, SEQ ID NO 55, SEQ ID NO 56 and SEQ ID NO 57 is considered linked to the sequence encoding PAX6, SEQ ID NO 9, SEQ ID NO 87, SEQ ID NO 88, SEQ ID NO 89, SEQ ID NO 90 and SEQ ID NO 91 is considered linked to the sequence encoding BARHL2, SEQ ID NO 10 is considered linked to the sequence encoding VCAN, SEQ ID NO 11 is considered linked to the sequence encoding VCAN, SEQ ID NO 12 is considered linked to the sequence encoding FAT4, SEQ ID NO 13 is considered linked to the sequence encoding ONECUT2, SEQ ID NO 14, SEQ ID NO 83 and SEQ ID NO 84 is considered linked to the sequence encoding LRAT, SEQ ID NO 15, SEQ ID NO 46, SEQ ID NO 47 and SEQ ID NO 48 is considered linked to the sequence encoding HAND2, SEQ ID NO 16 is considered linked to the sequence encoding FAM72D, SEQ ID NO 17 is considered linked to the sequence encoding EBF2, SEQ ID NO 18 is considered linked to the sequence encoding FAM19A4, SEQ ID NO 19 is considered linked to the sequence encoding NALCN, SEQ ID NO 20 is considered linked to the sequence encoding CD40, SEQ ID NO 21 is considered linked to the sequence encoding MEOX2, SEQ ID NO 22 is considered linked to the sequence encoding ZNF804B, SEQ ID NO 23 is considered linked to the sequence encoding GRIA4, SEQ ID NO 24, SEQ ID NO 85 and SEQ ID NO 86 is considered linked to the sequence encoding DLX5, SEQ ID NO 25, SEQ ID NO 58, SEQ ID NO 59, SEQ ID NO 60, SEQ ID NO 61 and SEQ ID NO 62 is considered linked to the sequence encoding SLC6A15, SEQ ID NO 26, SEQ ID NO 63, SEQ ID NO 64, SEQID NO 65, SEQID NO 66, SEQID NO 67, SEQ ID NO 68 and SEQ ID NO 69 is considered linked to the sequence encoding TBX5, SEQ ID NO 27 is considered linked to the sequence encoding SCAND3, SEQ ID NO 28 is considered linked to the sequence encoding FAM133A, SEQ ID NO 29 is considered linked to the sequence encoding NAP1 L3, SEQ ID NO 30 is considered linked to the sequence encoding SSTR2, SEQ ID NO 31 is considered linked to the sequence encoding NKX2-2, SEQ ID NO 32 is considered linked to the sequence encoding PRMT8, SEQ ID NO 33 is considered linked to the sequence encoding ITGA4, SEQ ID NO 34 is considered linked to the sequence encoding DPP10, SEQ ID NO 35 is considered linked to the sequence encoding DPP10, SEQ ID NO 36 is considered linked to the sequence encoding ADAMTS5, SEQ ID NO 37 is considered linked to the sequence encoding SNAP25, SEQ ID NO 38 is considered linked to the sequence encoding KCNA1, SEQ ID NO 39 is considered linked to the sequence encoding LRFN5, SEQ ID NO 40 is considered linked to the sequence encoding ZIC1, SEQ ID NO 41 is considered linked to the sequence encoding SEMA3D, SEQ ID NO 42 is considered linked to the sequence encoding TBCA, SEQ ID NO 43 is considered linked to the sequence encoding PCDH15, SEQ ID NO 44, SEQ ID NO 51 and SEQ ID NO 52 is considered linked to the sequence encoding mir129-2 and SEQ ID NO 45 is considered linked to the sequence encoding ZNF606.

### Biomarker for early screening

Several studies suggest that changes in the methylation status of the DNA molecule are already present in the "normal" mucosa in patients who have precancerous colorectal cancer, thus these changes in methylation status is present before any overt signs of the disease is visible.

The methylation status in the normal tissue from the patients with normal-appearing tissue was compared to the methylation status of tissue from patients with adenomas, early and late cancer. This has resulted in a group of differential methylated CpGs which may be used as biomarkers which can be used to screen stool DNA for precancerous changes before any overt changes is visible.

Differential methylation in at least on CpG in at least one of the at least one CpG rich region is indicative of the presence of adenomas before overt changes in the colorectal mucosa is visible.

In one embodiment of the present disclosure the differential methylation of at least one CpG in a CpG rich region selected from the group consisting of: SEQ ID NO: 93, SEQ ID NO 94, SEQ ID NO 95, SEQ ID NO 96, SEQ ID NO 97, SEQ ID NO 98, SEQ ID NO 99 and SEQ ID NO 100 may thus be used as an early biomarker for screening for adenomas before overt changes in the colorectal mucosa is visible. In particular, methylation of least one CpG in a CpG rich region selected from the group consisting of: SEQ ID NO: 93, SEQ ID NO 94, SEQ ID NO 95, SEQ ID NO 96, SEQ ID NO 97, SEQ ID NO 98, SEQ ID NO 99 and SEQ ID NO 100 is indicative of the presence of adenoma even before overt changes in the colorectal mucosa are visible.

In one embodiment, the determination of the methylation status of at least one CpG in SEQ ID NO: 92 is combined with the determination of the methylation status of at least one CpG present in the CpG rich region selected from the group consisting of: SEQ ID NO: 93, SEQ ID NO: 94, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 93 is combined with the determination of the methylation status of at least one CpG present in the CpG rich region selected from the group consisting of: SEQ ID NO: 92, SEQ ID NO: 94, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 94 is combined with the determination of the methylation status of at least one CpG present in the CpG rich region selected from the group consisting of: SEQ ID NO: 92, SEQ ID NO: 93, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 95 is combined with the determination of the methylation status of at least one CpG present in the CpG rich region selected from the group consisting of: SEQ ID NO: 92, SEQ ID NO: 93, SEQ ID NO: 94, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 96 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 97 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 98 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 99 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of at least one CpG in SEQ ID NO: 100 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 98, and SEQ ID NO: 99. Preferably methylation of at least half, such as of at least two of said CpG is indicative of an adenoma of the colon.

In one embodiment the methylation status of the CpG rich region of SEQ ID NO: 92 is determined by evaluating the methylation status of at least one CpG within the region of SEQ ID NO: 92, more preferably within the region +/- 150 base pairs from position 200 in SEQ ID NO: 92, even more preferably within the region +/- 100 base pairs from position 200 in SEQ ID NO: 92, even more preferably within the region +/- 50 base pairs from position 200 in SEQ ID NO: 92 for the presence of methylated CpGs. In a most preferred embodiment the methylation status of the CpG of position 200 of SEQ ID NO: 92 is determined.
If the at least one CpG within the region of SEQ ID NO: 92 is methylated, said methylation is indicative of the presence of an adenoma before overt signs are visible in the colorectal mucosa.

In one embodiment the methylation status of the CpG rich region of SEQ ID NO: 93 is determined by evaluating the methylation status of at least one CpG within the region of SEQ ID NO: 93, more preferably within the region +/- 200 base pairs from position 200 and/or within the region +/- 200 base pairs from position 251 and/or within the region +/- 200 base pairs from position 254 and/or within the region +/- 200 base pairs from position 258 and/or within the region +/- 200 base pairs from position 368 and/or within the region +/- 200 base pairs from position 389 in SEQ ID NO: 93, even more preferably within the region +/- 100 base pairs from position 200 and/or within the region +/- 100 base pairs from position 251 and/or within the region +/- 100 base pairs from position 254 and/or within the region +/- 100 base pairs from position 258 and/or within the region +/- 100 base pairs from position 368 and/or within the region +/- 100 base pairs from position 389 in SEQ ID NO: 93, even more preferably within the region +/- 50 base pairs from position 200 and/or within the region +/- 50 base pairs from position 251 and/or within the region +/- 50 base pairs from position 254 and/or within the region +/- 50 base pairs from position 258 and/or within the region +/- 50 base pairs from position 368 and/or within the region +/- 50 base pairs from position 389 in SEQ ID NO: 93 for the presence of methylated CpGs. In a most preferred embodiment the methylation status of the CpG of position 200 and/or position 251 and/or position 254 and/or position 258 and/or position 368 and/or position 389 of SEQ ID NO: 93 is determined.
If the at least one CpG within the region of SEQ ID NO: 93 is methylated, said methylation is indicative of the presence of an adenoma before overt signs are visible in the colorectal mucosa.

In one embodiment the methylation status of the CpG rich region of SEQ ID NO: 94 is determined by evaluating the methylation status of at least one CpG within the region of SEQ ID NO: 94, more preferably within the region +/- 200 base pairs from position 200 and/or within the region +/- 200 base pairs from position 495 and/or within the region +/- 200 base pairs from position 605 and/or within the region +/- 200 base pairs from position 1037 and/or within the region +/- 200 base pairs from position 1157 and/or within the region +/- 200 base pairs from position 2035 and/or within the region +/- 200 base pairs from position 2233 and/or within the region +/- 200 base pairs from position 4426 and/or within the region +/- 200 base pairs from position 6683 and/or within the region +/- 200 base pairs from position 7026 and/or within the region +/- 200 base pairs from position 7188 in SEQ ID NO: 94, even more preferably within the region +/- 100 base pairs from position 200 and/or within the region +/- 100 base pairs from position 495 and/or within the region +/- 100 base pairs from position 605 and/or within the region +/- 100 base pairs from position 1037 and/or within the region +/- 100 base pairs from position 1157 and/or within the region +/- 100 base pairs from position 2035 and/or within the region +/- 100 base pairs from position 2233 and/or within the region +/- 100 base pairs from position 4426 and/or within the region +/- 100 base pairs from position 6683 and/or within the region +/- 100 base pairs from position 7026 and/or within the region +/- 100 base pairs from position 7188 in SEQ ID NO: 94, even more preferably within the region +/- 50 base pairs from position 200 and/or within the region +/- 50 base pairs from position 495 and/or within the region +/- 50 base pairs from position 605 and/or within the region +/- 50 base pairs from position 1037 and/or within the region +/- 50 base pairs from position 1157 and/or within the region +/- 50 base pairs from position 2035 and/or within the region +/- 50 base pairs from position 2233 and/or within the region +/- 50 base pairs from position 4426 and/or within the region +/- 50 base pairs from position 6683 and/or within the region +/- 50 base pairs from position 7026 and/or within the region +/- 50 base pairs from position 7188 in SEQ ID NO: 94 for the presence of methylated CpGs. In a most preferred embodiment the methylation status of the CpG of position 200 and/or position 495 and/or position 605 and/or position 1037 and/or position 1157 and/or position 2035 and/or position 2233 and/or position 4426 and/or position 6683 and/or position 7026 and/or position 7188 of SEQ ID NO: 94 is determined.
If the at least one CpG within the region of SEQ ID NO: 94 is methylated, said methylation is indicative of the presence of an adenoma before overt signs are visible in the colorectal mucosa.

In one embodiment the methylation status of the CpG rich region of SEQ ID NO: 95 is determined by evaluating the methylation status of at least one CpG within the region of SEQ ID NO: 95, more preferably within the region +/- 200 base pairs from position 200 and/or within the region +/- 200 base pairs from position 32749 and/or within the region +/- 200 base pairs from position 39409 in SEQ ID NO: 95, even more preferably within the region +/- 100 base pairs from position 200 and/or within the region +/- 100 base pairs from position 32749 and/or within the region +/- 100 base pairs from position 39409 in SEQ ID NO: 95, even more preferably within the region +/- 50 base pairs from position 200 and/or within the region +/- 50 base pairs from position 32749 and/or within the region +/- 50 base pairs from position 39409 in SEQ ID NO: 95 for the presence of methylated CpGs. In a most preferred embodiment the methylation status of the CpG of position 200 and/or position 32749 and/or position 39409 of SEQ ID NO: 95 is determined.

If the at least one CpG within the region of SEQ ID NO: 95 is methylated, said methylation is indicative of the presence of an adenoma before overt signs are visible in the colorectal mucosa.

In one embodiment the methylation status of the CpG rich region of SEQ ID NO: 96 is determined by evaluating the methylation status of at least one CpG within the region of SEQ ID NO: 96, more preferably within the region +/- 200 base pairs from position 200 and/or within the region +/- 200 base pairs from position 1505 and/or within the region +/- 200 base pairs from position 8966 and/or within the region +/- 200 base pairs from position 12490 and/or within the region +/- 200 base pairs from position 12677 and/or within the region +/- 200 base pairs from position 12835 and/or within the region +/- 200 base pairs from position 12888 and/or within the region +/- 200 base pairs from position 13156 and/or within the region +/- 200 base pairs from position 13201 and/or within the region +/- 200 base pairs from position 14309 and/or within the region +/-200 base pairs from position 15814 and/or within the region +/- 200 base pairs from position 15950 and/or within the region +/- 200 base pairs from position 16288 and/or within the region +/- 200 base pairs from position 16351 in SEQ ID NO: 96, even more preferably within the region +/- 100 base pairs from position 200 and/or within the region +/- 100 base pairs from position 1505 and/or within the region +/- 100 base pairs from position 8966 and/or within the region +/- 100 base pairs from position 12490 and/or within the region +/- 100 base pairs from position 12677 and/or within the region +/- 100 base pairs from position 12835 and/or within the region +/- 100 base pairs from position 12888 and/or within the region +/- 100 base pairs from position 13156 and/or within the region +/- 100 base pairs from position 13201 and/or within the region +/-100 base pairs from position 14309 and/or within the region +/- 100 base pairs from position 15814 and/or within the region +/- 100 base pairs from position 15950 and/or within the region +/- 100 base pairs from position 16288 and/or within the region +/-100 base pairs from position 16351 in SEQ ID NO: 96, even more preferably within the region +/- 50 base pairs from position 200 and/or within the region +/- 50 base pairs from position 1505 and/or within the region +/- 50 base pairs from position 8966 and/or within the region +/- 50 base pairs from position 12490 and/or within the region +/- 50 base pairs from position 12677 and/or within the region +/- 50 base pairs from position 12835 and/or within the region +/- 50 base pairs from position 12888 and/or within the region +/- 50 base pairs from position 13156 and/or within the region +/- 50 base pairs from position 13201 and/or within the region +/- 50 base pairs from position 14309 and/or within the region +/- 50 base pairs from position 15814 and/or within the region +/- 50 base pairs from position 15950 and/or within the region +/- 50 base pairs from position 16288 and/or within the region +/- 50 base pairs from position 16351 in SEQ ID NO: 96 for the presence of methylated CpGs. In a most preferred embodiment the methylation status of the CpG of position 200 and/or position 1505 and/or position 8966 and/or position 12490 and/or position 12677 and/or position 12835 and/or position 12888 and/or position 13156 and/or position 13201 and/or position 14309 and/or position 15814 and/or position 15950 and/or position 16288 and/or position 16351 of SEQ ID NO: 96 is determined.
If the at least one CpG within the region of SEQ ID NO: 96 is methylated, said methylation is indicative of the presence of an adenoma before overt signs are visible in the colorectal mucosa.

In one embodiment the methylation status of the CpG rich region of SEQ ID NO: 97 is determined by evaluating the methylation status of at least one CpG within the region of SEQ ID NO: 97, more preferably within the region +/- 200 base pairs from position 200 and/or within the region +/- 200 base pairs from position 227 and/or within the region +/- 200 base pairs from position 376 and/or within the region +/- 200 base pairs from position 1418 and/or within the region +/- 200 base pairs from position 1641 and/or within the region +/- 200 base pairs from position 1656 and/or within the region +/- 200 base pairs from position 1747 in SEQ ID NO: 97, even more preferably within the region +/- 100 base pairs from position 200 and/or within the region +/- 100 base pairs from position 227 and/or within the region +/- 100 base pairs from position 376 and/or within the region +/- 100 base pairs from position 1418 and/or within the region +/- 100 base pairs from position 1641 and/or within the region +/- 100 base pairs from position 1656 and/or within the region +/-100 base pairs from position 1747 in SEQ ID NO: 97, even more preferably within the region +/- 50 base pairs from position 200 and/or within the region +/- 50 base pairs from position 227 and/or within the region +/-50 base pairs from position 376 and/or within the region +/- 50 base pairs from position 1418 and/or within the region +/- 50 base pairs from position 1641 and/or within the region +/- 50 base pairs from position 1656 and/or within the region +/- 50 base pairs from position 1747 in SEQ ID NO: 97 for the presence of methylated CpGs. In a most preferred embodiment the methylation status of the CpG of position 200 and/or position 227 and/or position 376 and/or position 1418 and/or position 1641 and/or position 1656 and/or position 1747 of SEQ ID NO: 97 is determined.

If the at least one CpG within the region of SEQ ID NO: 97 is methylated, said methylation is indicative of the presence of an adenoma before overt signs are visible in the colorectal mucosa.

In one embodiment the methylation status of the CpG rich region of SEQ ID NO: 98 is determined by evaluating the methylation status of at least one CpG within the region of SEQ ID NO: 98, more preferably within the region +/- 200 base pairs from position 200 and/or within the region +/- 200 base pairs from position 253 and/or within the region +/- 200 base pairs from position 392 and/or within the region +/- 200 base pairs from position 536 and/or within the region +/- 200 base pairs from position 966 and/or within the region +/- 200 base pairs from position 1153 in SEQ ID NO: 98, even more preferably within the region +/- 100 base pairs from position 200 and/or within the region +/- 100 base pairs from position 253 and/or within the region +/- 100 base pairs from position 392 and/or within the region +/- 100 base pairs from position 536 and/or within the region +/- 100 base pairs from position 966 and/or within the region +/- 100 base pairs from position 1153 in SEQ ID NO: 98, even more preferably within the region +/- 50 base pairs from position 200 and/or within the region +/- 50 base pairs from position 253 and/or within the region +/- 50 base pairs from position 392 and/or within the region +/- 50 base pairs from position 536 and/or within the region +/- 50 base pairs from position 966 and/or 1153 in SEQ ID NO: 98 for the presence of methylated CpGs. In a most preferred embodiment the methylation status of the CpG of position 200 and/or position 253 and/or position 392 and/or position 536 and/or position 966 and/or position 1153 of SEQ ID NO: 98 is determined.
If the at least one CpG within the region of SEQ ID NO: 98 is methylated, said methylation is indicative of the presence of an adenoma before overt signs are visible in the colorectal mucosa.

In one embodiment the methylation status of the CpG rich region of SEQ ID NO: 99 is determined by evaluating the methylation status of at least one CpG within the region of SEQ ID NO: 99, more preferably within the region +/- 200 base pairs from position 200 and/or within the region +/- 200 base pairs from position 381 and/or within the region +/- 200 base pairs from position 590 and/or within the region +/- 200 base pairs from position 813 and/or within the region +/- 200 base pairs from position 1039 and/or within the region +/- 200 base pairs from position 1084 in SEQ ID NO: 99, even more preferably within the region +/- 100 base pairs from position 200 and/or within the region +/- 100 base pairs from position 381 and/or within the region +/- 100 base pairs from position 590 and/or within the region +/- 100 base pairs from position 813 and/or within the region +/- 100 base pairs from position 1039 and/or within the region +/- 100 base pairs from position 1084 in SEQ ID NO: 99, even more preferably within the region +/- 50 base pairs from position 200 and/or within the region +/- 50 base pairs from position 381 and/or within the region +/- 50 base pairs from position 590 and/or within the region +/- 50 base pairs from position 813 and/or within the region +/- 50 base pairs from position 1039 and/or within the region +/- 50 base pairs from position 1084 in SEQ ID NO: 99 for the presence of methylated CpGs. In a most preferred embodiment the methylation status of the CpG of position 200 and/or position 381 and/or position 590 and/or position 813 and/or position 1039 and/or position 1084 of SEQ ID NO: 99 is determined.
If the at least one CpG within the region of SEQ ID NO: 99 is methylated, said methylation is indicative of the presence of an adenoma before overt signs are visible in the colorectal mucosa.

In one embodiment the methylation status of the CpG rich region of SEQ ID NO: 100 is determined by evaluating the methylation status of at least one CpG within the region of SEQ ID NO: 100, more preferably within the region +/- 200 base pairs from position 200 and/or within the region +/- 200 base pairs from position 270 and/or within the region +/- 200 base pairs from position 514 and/or within the region +/- 200 base pairs from position 2144 and/or within the region +/- 200 base pairs from position 3098 and/or within the region +/- 200 base pairs from position 3197 and/or within the region +/- 200 base pairs from position 3263 in SEQ ID NO: 100, even more preferably within the region +/- 100 base pairs from position 200 and/or within the region +/- 100 base pairs from position 270 and/or within the region +/- 100 base pairs from position 514 and/or within the region +/- 100 base pairs from position 2144 and/or within the region +/- 100 base pairs from position 3098 and/or within the region +/- 100 base pairs from position 3197 and/or within the region +/-100 base pairs from position 3263 in SEQ ID NO: 100, even more preferably within the region +/- 50 base pairs from position 200 and/or within the region +/- 50 base pairs from position 270 and/or within the region +/-50 base pairs from position 514 and/or within the region +/- 50 base pairs from position 2144 and/or within the region +/- 50 base pairs from position 3098 and/or within the region +/- 50 base pairs from position 3197 and/or within the region +/- 50 base pairs from position 3263 in SEQ ID NO: 100 for the presence of methylated CpGs. In a most preferred embodiment the methylation status of the CpG of position 200 and/or position 270 and/or position 514 and/or position 2144 and/or position 3098 and/or position 3197 and/or position 3263 of SEQ ID NO: 100 is determined.
If the at least one CpG within the region of SEQ ID NO: 100 is methylated, said methylation is indicative of the presence of an adenoma before overt signs are visible in the colorectal mucosa.

In one embodiment, the determination of the methylation status of the CpG at position 200 of SEQ ID NO: 92 is combined with the determination of the methylation status of at least one CpG present in the CpG rich region selected from the group consisting of: SEQ ID NO: 93, SEQ ID NO: 94, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 251 and/or position 254 and/or position 258 and/or position 368 and/or position 389 of SEQ ID NO: 93 is combined with the determination of the methylation status of at least one CpG present in the CpG rich region selected from the group consisting of: SEQ ID NO: 92, SEQ ID NO: 94, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 495 and/or position 605 and/or position 1037 and/or position 1157 and/or position 2035 and/or position 2233 and/or position 4426 and/or position 6683 and/or position 7026 and/or position 7188 of SEQ ID NO: 94 is combined with the determination of the methylation status of at least one CpG present in the CpG rich region selected from the group consisting of: SEQ ID NO: 92, SEQ ID NO: 93, SEQ ID NO: 95, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 32749 and/or position 39409 of SEQ ID NO: 95 is combined with the determination of the methylation status of at least one CpG present in the CpG rich region selected from the group consisting of: SEQ ID NO: 92, SEQ ID NO: 93, SEQ ID NO: 94, SEQ ID NO: 96, SEQ ID NO: 97, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 1505 and/or position 8966 and/or position 12490 and/or position 12677 and/or position 12835 and/or position 12888 and/or position 13156 and/or position 13201 and/or position 14309 and/or position 15814 and/or position 15950 and/or position 16288 and/or position 16351 of SEQ ID NO: 96 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 97, SEQ ID NO: 98, SEQ ID NO: 99, and SEQ ID NO: 100. Preferably methylation of at least half, such as of at least two of said CpG is indicative of an adenoma of the colon.
In one embodiment, the determination of the methylation status of the CpG at position 200 and/or position 227 and/or position 376 and/or position 1418 and/or position 1641 and/or position 1656 and/or position 1747 of SEQ ID NO: 97 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 98, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 253 and/or position 392 and/or position 536 and/or position 966 and/or position 1153 of SEQ ID NO: 98 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 99, and SEQ ID NO: 100. Preferably methylation of at least half, such as of at least two of said CpG is indicative of an adenoma of the colon.

In one embodiment, the determination of the methylation status of the CpG at position 200 and/or position 381 and/or position 590 and/or position 813 and/or position 1039 and/or position 1084 of SEQ ID NO: 99 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 98, and SEQ ID NO: 100. Preferably methylation of at least half, such as of at least two of said CpG is indicative of an adenoma of the colon.

In one embodiment, the determination of the methylation status of the CpG at position 200 and/or position 270 and/or position 514 and/or position 2144 and/or position 3098 and/or position 3197 and/or position 3263 of SEQ ID NO: 100 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 98, and SEQ ID NO: 99. Preferably methylation of at least half, such as of at least two of said CpG is indicative of an adenoma of the colon.

In one embodiment the methylation of a CpG rich region of the present disclosure is considered to be linked to the expression of a given gene. Accordingly methylation of said CpG rich region may influence the expression of said gene.

In one embodiment a CpG rich region according to the present disclosure is a CpG rich region which is linked to a gene selected from the group consisting of: RFC5, HOXA6, NR5A2, HOXC4, PITX2, SND1, OPLAH, FBLIM1 and OTX1.

Without being bound by theory SEQ ID NO 92 is considered linked to the sequence encoding RFC5, SEQ ID NO 93 is considered linked to the sequence encoding HOXA6, SEQ ID NO 94 is considered linked to the sequence encoding NR5A2, SEQ ID NO 95 is considered linked to the sequence encoding HOXC4, SEQ ID NO 96 is considered linked to the sequence encoding PITX2, SEQ ID NO 97 is considered linked to the sequence encoding SND1, SEQ ID NO 98 is considered linked to the sequence encoding OPLAH, SEQ ID NO 99 is considered linked to the sequence encoding FBLIM1 and SEQ ID NO 100 is considered linked to the sequence encoding OTX1,

### Determining transcriptional expression levels of the gene of interest

Preferred CpG rich regions according to the present invention are CpG rich regions which are linked to differential expression in adenomas contra normal tissue of the gene to which the CpG rich region is linked (see section on "Gene of interest"). Transcriptional expression levels of genes may in general be determined by detecting mRNA from the cells or sample of interest. The expression level of a given gene may be determined using any of the methods mentioned herein below. Transcriptional expression products, such as mRNA may be detected by any suitable technique known to the person skilled in the art.

### Extraction of RNA

RNA or protein can be isolated and assayed from a test sample using any techniques known in the art. They can for example be isolated from a fresh or frozen biopsy, from formalin-fixed tissue, from body fluids, such as blood, plasma, serum, or faeces.

Methods of isolating total mRNA are well known to those of skill in the art. In one embodiment, the total nucleic acid is isolated from a given sample using, for example, an acid guanidinium-phenol-chloroform extraction method and polyA mRNA is isolated by oligo dT column chromatography or by using (dT)n magnetic beads (see, e.g., Sambrook et al., Molecular Cloning: A Laboratory Manual (2nd ed.), Vols. 1-3, Cold Spring Harbor Laboratory, (1989), or Current Protocols in Molecular Biology, F. Ausubel et al., ed. Greene Publishing and Wiley-Interscience, New York (1987)).

The sample may be from tissue and/or body fluids, as defined in the section "Samples" herein above. Before analyzing the sample, e.g., on an oligonucleotide array, it will often be desirable to perform one or more sample preparation operations upon the sample. Typically, these sample preparation operations will include such manipulations as extraction of intracellular material, e.g., nucleic acids from whole cell samples, viruses, amplification of nucleic acids, fragmentation, transcription, labeling and/or extension reactions.
For a number of applications, it may be desirable to extract and separate messenger RNA from cells, cellular debris, and other contaminants. In general, such purification may take advantage of the poly-A tails on mRNA. In particular and as noted above, poly-T oligonucleotides may be immobilized within a chamber or channel to serve as affinity ligands for mRNA. Poly-T oligonucleotides may be immobilized upon a solid support incorporated within the chamber or channel, or alternatively, may be immobilized upon the surface(s) of the chamber or channel itself. Immobilization of oligonucleotides on the surface of the chambers or channels may be carried out by methods described herein including, e.g., oxidation and silanation of the surface followed by standard DMT synthesis of the oligonucleotides.

In operation, the lysed sample may be introduced to a high salt solution to increase the ionic strength for hybridization, whereupon the mRNA will hybridize to the immobilized poly-T. The mRNA bound to the immobilized poly-T oligonucleotides may then be washed free in a low ionic strength buffer. The poly-T oligonucleotides may be immobiliized upon porous surfaces, e.g., porous silicon, zeolites silica xerogels, scintered particles, or other solid supports.

Following sample preparation, the sample can be subjected to one or more different analysis operations. A variety of analysis operations may generally be performed, including size based analysis using, e.g., microcapillary electrophoresis, and/or sequence based analysis using, e.g., hybridization to an oligonucleotide array.

In the latter case, the nucleic acid sample may be probed using an array of oligonucleotide probes. Oligonucleotide arrays generally include a substrate having a large number of positionally distinct oligonucleotide probes attached to the substrate. These arrays may be produced using mechanical or light directed synthesis methods which incorporate a combination of photolithographic methods and solid phase oligonucleotide synthesis methods.

The basic strategy for light directed synthesis of oligonucleotide arrays is as follows. The surface of a solid support, modified with photosensitive protecting groups is illuminated through a photolithographic mask, yielding reactive hydroxyl groups in the illuminated regions. A selected nucleotide, typically in the form of a 3'-O-phosphoramidite-activated deoxynucleoside (protected at the 5' hydroxyl with a photosensitive protecting group), is then presented to the surface and coupling occurs at the sites that were exposed to light. Following capping and oxidation, the substrate is rinsed and the surface is illuminated through a second mask to expose additional hydroxyl groups for coupling. A second selected nucleotide (e.g., 5'-protected, 3'-O-phosphoramidite-activated deoxynucleoside) is presented to the surface. The selective deprotection and coupling cycles are repeated until the desired set of products is obtained. Since photolithography is used the process can be readily miniaturized to generate high density arrays of oligonucleotide probes. Furthermore, the sequence of the oligonucleotide at each site is known. Mechanical synthesis methods are similar to the light directed methods except involving mechanical direction of fluids for deprotection and addition in the synthesis steps.

For some embodiments, oligonucleotide arrays may be prepared having all possible probes of a given length. The hybridization pattern of the target sequence on the array may be used to reconstruct the target DNA sequence. Hybridization analysis of large numbers of probes can be used to sequence long stretches of DNA or provide an oligonucleotide array which is specific and complementary to a particular nucleic acid sequence. For example, in particularly preferred aspects, the oligonucleotide array will contain oligonucleotide probes which are complementary to specific target sequences and individual or multiple mutations of these. Such arrays are particularly useful in the diagnosis of specific disorders which are characterized by the presence of a particular nucleic acid sequence.

Following sample collection and nucleic acid extraction, the nucleic acid portion of the sample is typically subjected to one or more preparative reactions. These preparative reactions include in vitro transcription, labelling, fragmentation, amplification and other reactions. Nucleic acid amplification increases the number of copies of the target nucleic acid sequence of interest. A variety of amplification methods are suitable for use in the methods of the present invention, including for example, the polymerase chain reaction method or (PCR), the ligase chain reaction (LCR), self sustained sequence replication (3SR), and nucleic acid based sequence amplification (NASBA).

The latter two amplification methods involve isothermal reactions based on isothermal transcription, which produce both single stranded RNA (ssRNA) and double stranded DNA (dsDNA) as the amplification products in a ratio of approximately 30 or 100 to 1, respectively. As a result, where these latter methods are employed, sequence analysis may be carried out using either type of substrate, i.e. complementary to either DNA or RNA.

Frequently, it is desirable to amplify the nucleic acid sample prior to hybridization. One of skill in the art will appreciate that whatever amplification method is used, if a quantitative result is desired, care must be taken to use a method that maintains or controls for the relative frequencies of the amplified nucleic acids.

### Determining transcriptional levels by PCR-based methods

Polymerase Chain Reaction (PCR) is a well-known and well established technique to determine transcriptional products and therefore also a method that in one embodiment is used to determine the transcriptional expression level of the gene of interest, or part thereof according to the invention. One PCR-based method is LCR, which in one embodiment of the present invention may be used instead of PCR.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid.

Thus, in one embodiment, this disclosure provides for a method of optimizing a probe set for detection of a particular gene. Generally, this method involves providing a high density array containing a multiplicity of probes of one or more particular length(s) that are complementary to sub-sequences of the mRNA transcribed by the target gene. In one embodiment the high density array may contain every probe of a particular length that is complementary to a particular mRNA. The probes of the high density array are then hybridized with their target nucleic acid alone and then hybridized with a high complexity, high concentration nucleic acid sample that does not contain the targets complementary to the probes. Thus, for example, where the target nucleic acid is an RNA, the probes are first hybridized with their target nucleic acid alone and then hybridized with RNA made from a cDNA library where the sense of the hybridized RNA is opposite that of the target nucleic acid (to insure that the high complexity sample does not contain targets for the probes). Those probes that show a strong hybridization signal with their target and little or no cross-hybridization with the high complexity sample are preferred probes for use in the high density arrays of this disclosure.

PCR amplification generally involves the use of one strand of the target nucleic acid sequence as a template for producing a large number of complements to that sequence. Generally, two primer sequences complementary to different ends of a segment of the complementary strands of the target sequence hybridize with their respective strands of the target sequence, and in the presence of polymerase enzymes and nucleoside triphosphates, the primers are extended along the target sequence. The extensions are melted from the target sequence and the process is repeated, this time with the additional copies of the target sequence synthesized in the preceding steps. PCR amplification typically involves repeated cycles of denaturation, hybridization and extension reactions to produce sufficient amounts of the target nucleic acid. The first step of each cycle of the PCR involves the separation of the nucleic acid duplex formed by the primer extension. Once the strands are separated, the next step in PCR involves hybridizing the separated strands with primers that flank the target sequence. The primers are then extended to form complementary copies of the target strands. For successful PCR amplification, the primers are designed so that the position at which each primer hybridizes along a duplex sequence is such that an extension product synthesized from one primer, when separated from the template (complement), serves as a template for the extension of the other primer. The cycle of denaturation, hybridization, and extension is repeated as many times as necessary to obtain the desired amount of amplified nucleic acid.

In PCR methods, strand separation is normally achieved by heating the reaction to a sufficiently high temperature for a sufficient time to cause the denaturation of the duplex but not to cause an irreversible denaturation of the polymerase. Typical heat denaturation involves temperatures ranging from about 80 degrees Celsius to 105 degrees Celsius for times ranging from seconds to minutes. Strand separation, however, can be accomplished by any suitable denaturing method including physical, chemical, or enzymatic means. Strand separation may be induced by a helicase, for example, or an enzyme capable of exhibiting helicase activity.

Reverse transcription polymerase chain reaction (RT-PCR) is a variant of polymerase chain reaction (PCR). In RT-PCR an RNA strand is first reverse transcribed into its DNA complement (complementary DNA, or cDNA) using the enzyme reverse transcriptase, and the resulting cDNA is amplified using traditional PCR.

In one preferred embodiment the transcriptional expression level of the gene of interest is determined by RT-PCR.

In addition to PCR and IVT reactions, the methods of the present invention are also applicable to a number of other reaction types, e.g., reverse transcription, nick translation, and the like.

The nucleic acids in a sample will generally be labelled to facilitate detection in subsequent steps. Labelling may be carried out during the amplification, in vitro transcription or nick translation processes. In particular, amplification, in vitro transcription or nick translation may incorporate a label into the amplified or transcribed sequence, either through the use of labelled primers or the incorporation of labelled dNTPs into the amplified sequence.
Hybridization between the sample nucleic acid and the oligonucleotide probes upon the array is then detected, using, e.g., epifluorescence confocal microscopy. Typically, sample is mixed during hybridization to enhance hybridization of nucleic acids in the sample to nucleic acid probes on the array.

In some cases, hybridized oligonucleotides may be labelled following hybridization. For example, where biotin labeled dNTPs are used in, e.g. amplification or transcription, streptavidin linked reporter groups may be used to label hybridized complexes. Alternatively, the nucleic acids in the sample may be labelled following amplification. Post amplification labelling typically involves the covalent attachment of a particular detectable group upon the amplified sequences. Suitable labels or detectable groups include a variety of fluorescent or radioactive labelling groups well known in the art. These labels may also be coupled to the sequences using methods that are well known in the art.

Methods for detection of the transcript of the gene of interest or part thereof depend upon the label selected. A fluorescent label is preferred because of its extreme sensitivity and simplicity. Standard labelling procedures are used to determine the positions where interactions between a sequence and a reagent take place. For example, if a target sequence is labelled and exposed to a matrix of different probes, only those locations where probes do interact with the target will exhibit any signal.

Alternatively, other methods may be used to scan the matrix to determine where interaction takes place. Of course, the spectrum of interactions may be determined in a temporal manner by repeated scans of interactions which occur at each of a multiplicity of conditions. However, instead of testing each individual interaction separately, a multiplicity of sequence interactions may be simultaneously determined on a matrix.

Means of detecting labelled target (sample) nucleic acids hybridized to the probes of the high density array are known to those of skill in the art. Thus, for example, where a colorimetric label is used, simple visualization of the label is sufficient. Where a radioactive labelled probe is used, detection of the radiation (e.g with photographic film or a solid state detector) is sufficient.

In a preferred embodiment, however, the target nucleic acids are labelled with a fluorescent label and the localization of the label on the probe array is accomplished with fluorescent microscopy. The hybridized array is excited with a light source at the excitation wavelength of the particular fluorescent label and the resulting fluorescence at the emission wavelength is detected. In a particularly preferred embodiment, the excitation light source is a laser appropriate for the excitation of the fluorescent label.

The target polynucleotide may be labelled by any of a number of convenient detectable markers. A fluorescent label is preferred because it provides a very strong signal with low background. It is also optically detectable at high resolution and sensitivity through a quick scanning procedure. Other potential labelling moieties include, radioisotopes, chemiluminescent compounds, labelled binding proteins, heavy metal atoms, spectroscopic markers, magnetic labels, and linked enzymes.
Another method for labelling may bypass any label of the target sequence. The target may be exposed to the probes, and a double strand hybrid is formed at those positions only. Addition of a double strand specific reagent will detect where hybridization takes place. An intercalative dye such as ethidium bromide may be used as long as the probes themselves do not fold back on themselves to a significant extent forming hairpin loops. However, the length of the hairpin loops in short oligonucleotide probes would typically be insufficient to form a stable duplex.

Suitable chromogens will include molecules and compounds which absorb light in a distinctive range of wavelengths so that a color may be observed, or emit light when irradiated with radiation of a particular wave length or wave length range, e.g., fluorescers. Biliproteins, e.g., phycoerythrin, may also serve as labels.

A wide variety of suitable dyes are available, being primarily chosen to provide an intense color with minimal absorption by their surroundings. Illustrative dye types include quinoline dyes, triarylmethane dyes, acridine dyes, alizarine dyes, phthaleins, insect dyes, azo dyes, anthraquinoid dyes, cyanine dyes, phenazathionium dyes, and phenazoxonium dyes.

Desirably, fluorescers should absorb light above about 300 nm, preferably about 350 nm, and more preferably above about 400 nm, usually emitting at wavelengths greater than about 10 nm higher than the wavelength of the light absorbed. It should be noted that the absorption and emission characteristics of the bound dye may differ from the unbound dye. Therefore, when referring to the various wavelength ranges and characteristics of the dyes, it is intended to indicate the dyes as employed and not the dye which is unconjugated and characterized in an arbitrary solvent.

Fluorescers are generally preferred because by irradiating a fluorescer with light, one can obtain a plurality of emissions. Thus, a single label can provide for a plurality of measurable events.

Detectable signal may also be provided by chemiluminescent and bioluminescent sources. Chemiluminescent sources include a compound which becomes electronically excited by a chemical reaction and may then emit light which serves as the detectible signal or donates energy to a fluorescent acceptor. A diverse number of families of compounds have been found to provide chemiluminescence under a variety of conditions. One family of compounds is 2,3-dihydro-1,-4-phthalazinedione. The most popular compound is luminol, which is the 5-amino compound. Other members of the family include the 5-amino-6,7,8-trimethoxy- and the dimethylamino>ca!benz analog. These compounds can be made to luminescence with alkaline hydrogen peroxide or calcium hypochlorite and base. Another family of compounds is the 2,4,5-triphenylimidazoles, with lophine as the common name for the parent product. Chemiluminescent analogs include para-dimethylamino and -methoxy substituents. Chemiluminescence may also be obtained with oxalates, usually oxalyl active esters, e.g., p-nitrophenyl and a peroxide, e.g., hydrogen peroxide, under basic conditions.

Alternatively, luciferins may be used in conjunction with luciferase or lucigenins to provide bioluminescence.

Spin labels are provided by reporter molecules with an unpaired electron spin which can be detected by electron spin resonance (ESR) spectroscopy. Exemplary spin labels include organic free radicals, transitional metal complexes, particularly vanadium, copper, iron, and manganese, and the like. Exemplary spin labels include nitroxide free radicals.

In addition, amplified sequences may be subjected to other post amplification treatments. For example, in some cases, it may be desirable to fragment the sequence prior to hybridization with an oligonucleotide array, in order to provide segments which are more readily accessible to the probes, which avoid looping and/or hybridization to multiple probes. Fragmentation of the nucleic acids may generally be carried out by physical, chemical or enzymatic methods that are known in the art.

Following the various sample preparation operations, the sample will generally be subjected to one or more analysis operations. Particularly preferred analysis operations include, e.g. sequence based analyses using an oligonucleotide array and/or size based analyses using, e.g. microcapillary array electrophoresis.

In some embodiments it may be desirable to provide additional or alternative means for analyzing the nucleic acids from the sample

Microcapillary array electrophoresis generally involves the use of a thin capillary or channel which may or may not be filled with a particular separation medium. Electrophoresis of a sample through the capillary provides a size based separation profile for the sample. Microcapillary array electrophoresis generally provides a rapid method for size based sequencing, PCR product analysis and restriction fragment sizing. The high surface to volume ratio of these capillaries allows for the application of higher electric fields across the capillary without substantial thermal variation across the capillary, consequently allowing for more rapid separations. Furthermore, when combined with confocal imaging methods these methods provide sensitivity in the range of attomoles, which is comparable to the sensitivity of radioactive sequencing methods.

In many capillary electrophoresis methods, the capillaries e.g. fused silica capillaries or channels etched, machined or molded into planar substrates, are filled with an appropriate separation/sieving matrix. Typically, a variety of sieving matrices are known in the art may be used in the microcapillary arrays. Examples of such matrices include, e.g. hydroxyethyl cellulose, polyacrylamide and agarose. Gel matrices may be introduced and polymerized within the capillary channel. However, in some cases this may result in entrapment of bubbles within the channels which can interfere with sample separations. Accordingly, it is often desirable to place a preformed separation matrix within the capillary channel(s), prior to mating the planar elements of the capillary portion. Fixing the two parts, e.g. through sonic welding, permanently fixes the matrix within the channel. Polymerization outside of the channels helps to ensure that no bubbles are formed. Further, the pressure of the welding process helps to ensure a void-free system.

In one embodiment preferred CpG rich regions according to the present invention are CpG rich regions which are linked to differential expression in adenomas contra normal tissue of the gene to which the CpG rich region is linked (see section on "Gene of interest" herein above). Transcriptional expression products from the gene of interest may be detected as indications of adenomas in the sample tested, and accordingly be used for diagnosing and predicting adenomas in an individual. The transcriptional expression product of the gene of interest may be detected in either a tissue sample as such, or in a body fluid sample, such as blood, serum, plasma and/or faeces of the individual (see section on "Samples" herein above).

### Determining the translational expression level of the gene of interest

Preferred CpG rich regions according to the present invention are CpG rich regions which are linked to differential expression in adenomas contra normal tissue of the gene to which the CpG rich region is linked (see section on "Gene of interest"). In one embodiment of the present invention a decreased transcriptional and/or translational expression level of the gene of interest in a sample is indicative of the presence of adenomas.

### Methods for determining translational expression levels

The translational expression products such as peptides and proteins may be detected by any suitable technique or method known to the person skilled in the art.

The translational expression level of the gene of interest or part thereof may preferably be detected by means of specific antibodies directed to the protein product, such as immunofluorescent and/or immunohistochemical staining of the tissue.

Thus, in a preferred embodiment the translational expression level of the gene of interest is determined by immunohistochemical analysis.

Immunohistochemical localization of expressed protein may be carried out by immunostaining of tissue sections from for example tissue samples such as a biopsy.

In yet another embodiment the protein or part thereof may be detected by means of conventional enzyme assays, such as ELISA methods.

Furthermore, the protein or part thereof may be detected by means of peptide/protein chips capable of specifically binding the peptides and/or proteins assessed. Thereby an expression pattern may be obtained.

Possible translational expression products from the gene of interest linked to the CpG rich region of the invention (see section "Gene of interest" herein above) may be detected as indications of presence of adenomas in the sample tested, and accordingly may be used for diagnosing and predicting adenomas in an individual. The translational expression products of the gene of interest may be detected in either a tissue sample as such, or in a body fluid sample, such as blood, serum, plasma and/or faeces of the individual (see section "Samples" herein above). Preferably, the translational level is measured in a body fluid sample, preferably a faecal sample.

### Examples

### Example 1 - Identification of genes of interest

### Materials for DNA Methylation Microarray.

Ten samples of normal tissue and ten of adenomas were analyzed. Tissue samples were collected at the University Hospital of Birmingham, Queen Elizabeth Hospital in accordance with institutional policies. All patients provided written informed consent. The normal samples were taken from adjacent normal tissue from ten patients with cancer (average age 70 years). The tissue samples were stored in liquid nitrogen and then frozen within 10 minutes after they were collected.

The ten adenomas were selected based on them being "high risk" and matching ages (average age 72 years). There should be a high proportion of adenoma in the adenoma sample, mainly because of the way they are harvested.

| NimbleGen ID | Polyp risk | Pathology details | Polyp size (mm) | Localisation |
|---|---|---|---|---|
| A1 | High | TVA with low-grade dysplasia | 34x23x15 mm | Recto sigmoid |
| A2 | High | Tubular adenoma with moderate dysplasia | 15 mm | Recto sigmoid |
| A3 | High | Several tubular adenomas with low grade dysplasia | 10 mm | Recto sigmoid |
| A4 | High | Multiple (>20) TVA with low-grade dysplasia | 15 mm | Recto sigmoid |
| A5 | High | Tubular adenoma with high grade (severe) dysplasia | 7 mm | Recto sigmoid |
| A6 | High | TVA with low-grade dysplasia | 14 x 10 x 7 mm | Recto sigmoid |
| A7 | High | Villous adenoma displaying low-grade dysplasia | | Recto sigmoid |
| A8 | High | TVA with up to high-grade (severe) dysplasia | 15 mm | Recto sigmoid |
| A9 | High | TVA displaying low grade dysplasia | 10 mm | Recto sigmoid |
| A10 | High | TVA showing moderate dysplasia | 17 mm | Recto sigmoid |

### Methods for analysis data from the DNA Methylation Microarray.

The NimbleGen Human DNA Methylation 385K RefSeq Promoter Array that was designed based on the HG18 genome release was used in the present example. The array contains 385 019 probes with lengths of between 50-75 bp spaced in 100 bp steps along the upstream promoter regions of well-characterized RefSeq genes. Signal intensity data was extracted from the scanned images of the array using NimbleScan (Nimblegen).

Genomic DNA was extracted from the ten adenomas and normal tissue samples using Qiagen DNeasy kit. After step 2 in the manufacturer's protocol we added 4 µl RNaseA (100/ml), mixed thoroughly and incubated at 37°C for 30 min before continuing with step 3 of the manufacturer's protocol.

NimbleGen's DNA Methylation Microarray Services require 4 µg of DNA sample. First the DNA samples were fragmented by Diagenode's Bioruptor and then the size was controlled using Agilent's Bilanalyser. Enrichment of methylated DNA fragments was preformed as a technical duplicate using Invitrogen's MethylMiner Kit. A qPCR-based test was preformed to control the enrichment on the pooled replicates. Amplification was done using Sigma's Whole Genome Amplification kit (WGA1).

QPCR-based quality control of enrichment was preformed. The enriched and input samples were labeled with Cy5/Cy3. And then a quality control of yield and dye incorporation rates was done using Nanodrop. The samples were hybridized, washed and scanned onto NimbleGen arrays.

Data extraction and primary data analysis were done by NimbleGen's NimbleScan software. The extended data analysis is based on the peak files that have been obtained for each hybridization when applying the standard setting. Since the starting position of one particular peak might differ between samples it was important to make the peak files comparable among all samples. This was achieved by assigning peak in certain consistent windows of 300bp size.

Each feature on the array has a corresponding scaled log₂-ratio. This is the ratio of the signal intensities for the enriched and input samples that were co-hybridized to the array. The log₂-ratio is computed and scaled to center the ratio data around zero. Scaling is performed by subtracting the bi-weight mean for the log₂-ratio values for all features on the array from each log₂-ratio value.

As mentioned, the results from the Nimblegen array gave Log2-values for all the probe-positions present in the array. All the probes in each CpG rich region were then gathered using the pivot function in Microsoft excel. This generated data from 33.816 sequences mainly CpG rich regions.

The mean Log2-value from all 10 samples of normal tissue and adenomas was calculated. A threshold value at 0.2 in difference between mean values in normal tissue and adenomas was set, indicating that an increase in methylation in the CpG rich region is more than 0.2 in the log2 scale.
Level of methylation in the DNA rises when the normal mucosa changes to adenomas. And if the adenoma progress to carcinoma, the level most likely goes down again. A threshold on the log2 scale was set to be below -0.09 for normal tissue.

In order to have high sensitivity and selectivity in the test the raise in methylation from normal tissue to pre-malignant adenomas should be as high as possible. For that reason we also analyzed data with a threshold in difference between normal tissue and adenomas greater than 0.4. The main purpose in getting a high difference in methylation is to reduce the number of overlapping samples between normal tissue and adenomas. Overlapping samples leads to problems in determining which samples are normal and which are pre-malignant.

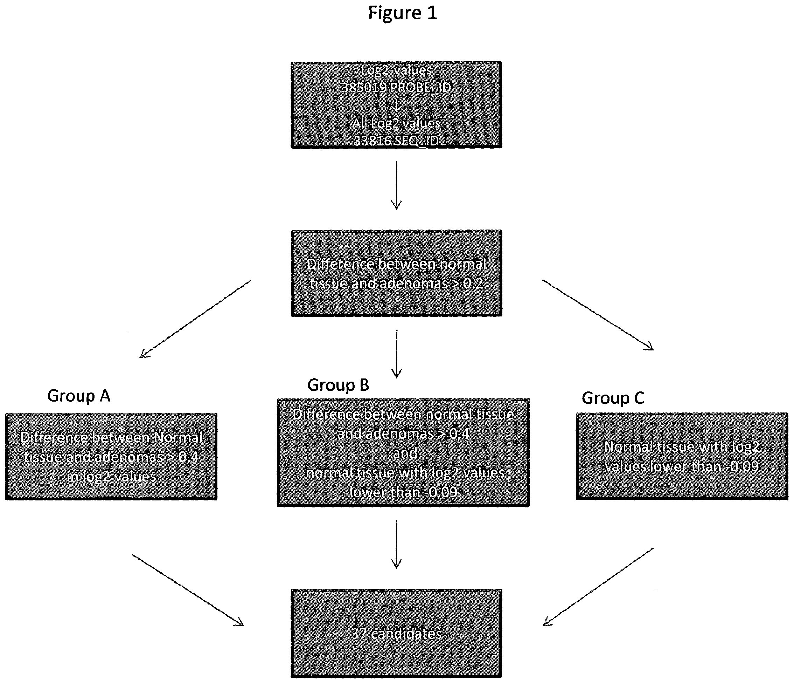

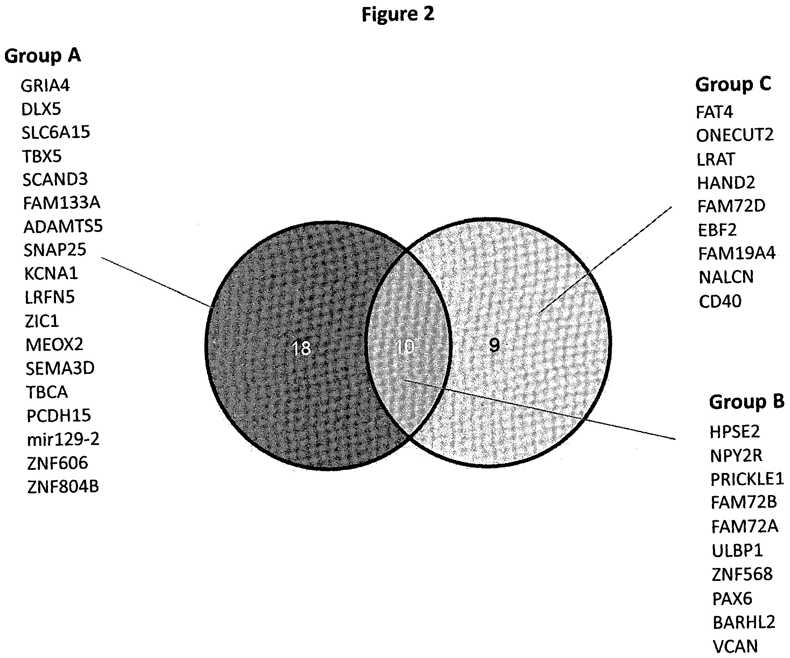

A Mann Whitney two-tailed t-test was used on all data. Only highly significant (P < 0.001, two-tailed test) results passed for further analysis. By setting the threshold as described above we secure a high sensitivity and specificity. Sensitivity is an expression of how well the test is to detect people with high risk of colorectal cancer. And specificity is related to the accuracy of classifying people with no increased risk. See figure 1 and 2.

### Result

Several differentially methylated regions (DMRs), promoters and CpG rich regions, that are hypermethylated in adenomas compared to normal tissue/mucosa were identified using the method described above. CpG rich regions are regions of the genome that have a high concentration of CpG sites. CpG rich regions typically occur at or near the transcription start site of genes, particularly housekeeping genes. When a CpG rich region is methylated, the related gene will not be translated and the expression of that particular gene will decrease. If a tumor suppressor gene (TSG) is lost or reduced, the cell is more vulnerable to developed into a cancer cell.

The relation between a specific CpG rich region and genes located nearby is not always clear as some CpG rich regions affect expression of genes that are placed far from its location. The following DMRs was identified:
Position on array: chr1:90.961.507-90.962.208 (SEQ ID NO: 9)
   Position on array: chr1:120.637.484-120.640.914 (SEQ ID NO: 4)
   Position on array: chr1:204.302.067-204.305.486 (SEQ ID NO: 5)
   Position on array: chr1_random:870.183-873.603 (SEQ ID NO: 16)
Position on array: chr2:115.136.114-115.136.815 (SEQ ID NO: 34)
   Position on array: chr2:115.635.207-115.637.235 (SEQ ID NO: 35)
   Position on array: chr2:182.029.063-182.031.274 (SEQ ID NO: 33)
Position on array: chr3:69.063.470-69.065.201 (SEQ ID NO: 18)
   Position on array: chr3:148.613.536-148.614.237 (SEQ ID NO: 40)
Position on array: chr4:126.455.345-126.458.380 (SEQ ID NO: 12)
   Position on array: chr4:155.883.162-155.885.283 (SEQ ID NO: 14)
   Position on array: chr4:156.348.430-156.349.659 (SEQ ID NO: 2)
   Position on array: chr4:174.679.881-174.680.582 (SEQ ID NO: 15)
Position on array: chr5:77.176.134-77.176.834 (SEQ ID NO: 42)
   Position on array: chr5:82.802.538-82.803.538 (SEQ ID NO: 11)
   Position on array: chr5:82.804.143-82.805.024 (SEQ ID NO: 10)
Position on array: chr6:28.710.703-28.711.403 (SEQ ID NO: 27)
   Position on array: chr6:150.326.035-150.328.208 (SEQ ID NO: 6)
Position on array: chr7:15.692.633-15.693.633 (SEQ ID NO: 21)
   Position on array: chr7:84.652.775-84.654.178 (SEQ ID NO: 41)
   Position on array: chr7:88.225.888-88.227.219 (SEQ ID NO: 22)
Position on array: chr7:96.491.403-96.492.879 (SEQ ID NO: 24)
Position on array: chr8:25.956.479-25.961.759 (SEQ ID NO: 17)

Position on array: chr10:57.060.349-57.061.221 (SEQ ID NO: 43)
   Position on array: chr10:100.983.644-100.984.344 (SEQ ID NO: 1)
Position on array: chr11:31.782.319-31.783.543 (SEQ ID NO: 8)
   Position on array: chr11:104.986.134-104.987.134 (SEQ ID NO: 23)
   Position on array: chr11:43.559.106-43.559.806 (SEQ ID NO: 44)

Position on array: chr12:3.469.885-3.473.269 (SEQ ID NO: 32)
   Position on array: chr12:4.888.846-4.891.432 (SEQ ID NO: 38)
   Position on array: chr12:41.269.099-41.270.851 (SEQ ID NO: 3)
   Position on array: chr12:83.829.092-83.830.251 (SEQ ID NO: 25)
   Position on array: chr12:113.328.072-113.329.072 (SEQ ID NO: 26)

Position on array: chr13:100.866.117-100.867.614 (SEQ ID NO: 19)
Position on array: chr14:41.146.293-41.147.293 (SEQ ID NO: 39)
Position on array: chr17:68.671.954-68.673.945 (SEQ ID NO: 30)
Position on array: chr18:53.254.152-53.259.851 (SEQ ID NO: 13)
Position on array: chr19:42.155.788-42.156.489 (SEQ ID NO: 7)
   Position on array: chr19:63.212.451-63.213.151 (SEQ ID NO: 45)
Position on array: chr20:10.146.135-10.147.676 (SEQ ID NO: 37)
   Position on array: chr20:21.433.932-21.444.714 (SEQ ID NO: 31)
   Position on array: chr20:44.179.512-44.180.698 (SEQ ID NO: 20)
Position on array: chr21:27.259.727-27.262.108 (SEQ ID NO: 36)
Position on array: chrX: 92.814.972-92.816.064 (SEQ ID NO: 28)
Position on array: chrX: 92.814.972-92.816.064 (SEQ ID NO: 29)

### Example 2 - Illumina Infinium 450K Methylation assay

The Illumina Infinium 450K Methylation assay requires only 500ng of DNA.

DNA from all 59 tissue samples were extracted using the QIAamp DNA Mini kit. Initially the quality of the DNA samples was checked using Nanodrop. Subsequently the DNA was treated with sodium bisulfite using the EZ DNA MethylationTM Kit. Bisulfite treatment of DNA was conducted immediately prior to conducting the analysis with the Infinium 450K breadchip, since bisulfite treated DNA is more fragile. Bisulfite treatment of DNA changes unmethylated cytosines to urasil but leaves methylated cytosines unchanged. The bisulfite-converted DNA samples were then denatured and amplified for 22 hours at 37°C of the. The amplified DNA was then enzymatically fragmented to give it an appropriate length for the subsequent hybridization. The fragmented DNA was precipitated and the supernatant is removed so that only the pure DNA is left. The precipitated DNA was resuspended in a hybridization solution and hybridizated on a MSA4 plate for 1 hour at 48°C.

The DNA samples were then placed on the beadchip and incubated at 48°C for 16-24 hours. During this step the DNA fragments will hybridize to the beads, which have a complementary sequence. Then the beadchip was washed.

Due to the oligo designs the single base extension of oligonucleotides on the beads takes place corresponding to the potentially methylated base. If the base at this position in the sample DNA is unmethylated the bisulfite treatment will have converted cytosines to uracil (U). And if the base is methylated the cytosine will remain unchanged (C). The base that is added at the single-base extension may therefore be either adenine (A), labeled with biotin or guanine (G), labeled with dinitrophenyl. The extended labelled bases will subsequently acquire fluorescence labeling since red labeled streptavidin is attached to the biotin and green labeled anti dinitrophenyl is attached to dinitropheny.

After wash the beadchip was coated with a protective film and dried in vacuo. The beadchip was subsequently scanned on the HiScanSQ reader. GenomeStudio™ Methylation Module version 1.8 (Illumina Inc., San Diego, CA, 2012) in order to calculate the methylation level at each CpG as the beta-value (β=intensity of the methylated allele (M) /(intensity of the unmethylated allele (U) + intensity of the methylated allele (M) + 100)) (Bibikova et al. 288-95). Beta values were then transformed to obtain the logratio, defined as log [β/(1-β)]. Raw β-values were extracted from GenomeStudio™ Methylation Module after being background substracted and normalized to eliminate batch effects.

The methylation level at each CpG was compared between different tissue samples. 10 tissue samples from normal mucosa from healthy individuals were examined (NN). 8 tissue samples from normal mucosa from patients with adenomas were examined (NP). 9 tissue samples from adenomas were examined (PN). 8 tissue samples from normal mucosa from patients with early stage colon cancer were examined (NE). 8 tissue samples from early stage colon cancer were examined (EN). 8 tissue samples from normal mucosa from patients with late stage colon cancer were examined (NL). 8 tissue samples from late stage colon cancer were examined (LN).
The methylation status was then compared in the following combinations: NP and PN was compared, NP and EN was compared and NP and LN was compared.
Furthermore, the results from the NN group were compared to the six other groups in the following combinations: NN and NP were compared, NN and NE were compared, NN and NL were compared, NN and PN were compared, NN and EN were compared and NN and LN were compared.

Several differentially methylated regions (DMRs), promoters and CpG rich regions, that are hypermethylated in adenomas compared to normal tissue/mucosa were identified using the method described above. The identified differentially methylated CpGs may be used to evaluate whether a given patient have an adenoma in the colon.
The differentially methylated regions found when comparing the normal mucosa from healthy individual with the "normal looking" mucosa from a patient with adenoma, early stage colon cancer or late stage colon cancer and be used as biomarkers for early screening giving indications of the presence/increased likelihood of adenomas even before overt signs of the adenomas is present in the colorectal mucosa.

## Claims

1. A method of diagnosis comprising determining the methylation status of at least one CpG within a CpG rich region in SEQ ID NO: 98, and wherein the method comprises the step of determining the methylation status of at least one CpG within said CpG rich region in genomic DNA of a sample of an individual at risk of acquiring an adenoma in the colon or rectum, and
wherein methylation of said at least one CpG is indicative of the presence of an adenoma of the colon in said individual.

2. The method according to claim 1, wherein the presence of methylation of said methylated CpG rich regions is used to predict the course of the disease in said individual.

3. The method according to claim 2, wherein the presence of methylation of said methylated CpG rich regions is used to predict the susceptibility to adenomas of the individual.

4. The method of claim 2, wherein the determination of the methylation status of at least one CpG in SEQ ID NO: 98 is combined with the determination of the methylation status of at least one CpG present in the CpG rich region selected from the group consisting of: SEQ ID NO: 92, SEQ ID NO: 93, SEQ ID NO: 94, SEQ ID NO: 95, SEQ ID NO: 96, SEQ ID NO: 97, SEQ ID NO: 99, and SEQ ID NO: 100.

5. The method according to claim 1 further comprising determining the methylation status of at least one CpG within a CpG rich region,
wherein said CpG rich region is selected from the group consisting of SEQ ID NO:27, SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, and SEQ ID NO: 91 or a sequence having at least 90% sequence identity thereto.

6. The method according to claim 5 wherein the CpG rich region consists of a continuous stretch of at the most 300 base pairs of a sequence selected from the group consisting of SEQ ID NO:27, SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 or a sequence at least 90% identical thereto.

7. The method according to claim 6, wherein the CpG rich region consist of a continuous stretch of at the most 300 base pairs of a sequence selected from the group consisting of SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38, and SEQ ID NO: 44 or a sequence at least 90% identical thereto.

8. The method according to claim 6, wherein at least one CpG rich region is associated with a gene selected from the group consisting of SCAND3, NPY2R, PRICKLE1, FAM72B, FAM72A, ULBP1, ZNF568, BARHL2, FAT4, FAM72D, EBF2, FAM19A4, NALCN, CD40, MEOX2, ZNF804B, DLX5, FAM133A, NKX2-2, PRMT8, DPP10, SNAP25, KCNA1, ZIC1, SEMA3D, TBCA, PCDH15, mir129-2 and ZNF606.

9. The method according to claim 6, wherein methylation status of at least 2 CpG's within one CpG rich region is determined, wherein methylation of both of said CpG's is indicative of the presence of an adenoma of the colon.

10. The method according to any one of the preceding claims, wherein the methylation status is determined by Multiplex Ligation-dependent Probe Amplification (MLPA) or PCR based method/LCR based method .

11. The method according to any one of the preceding claims, wherein the sample is a faecal sample or a blood sample.

12. The method according to any one of claims 1 to 12, wherein the sample is a biopsy of the colon, said biopsy containing normal mucosa and/or affected mucosa.

## Patentansprüche

1. Diagnoseverfahren, umfassend ein Bestimmen des Methylierungsstatus von mindestens einem CpG in einer CpG-reichen Region in SEQ ID NO: 98, und wobei das Verfahren den Schritt eines Bestimmens des Methylierungsstatus von mindestens einem CpG in der CpG-reichen Region in genomischer DNA einer Probe von einem Individuum beinhaltet, bei dem das Risiko besteht, dass es ein Adenom im Kolon oder Rektum bekommt, und
wobei eine Methylierung des mindestens einen CpG ein Hinweis auf das Vorhandensein eines Adenoms des Kolons bei dem Individuum ist.

2. Verfahren nach Anspruch 1, wobei das Vorhandensein einer Methylierung der methylierten CpG-reichen Regionen verwendet wird, um den Verlauf der Krankheit bei dem Individuum vorherzusagen.

3. Verfahren nach Anspruch 2, wobei das Vorhandensein einer Methylierung der methylierten CpG-reichen Regionen verwendet wird, um die Anfälligkeit auf Adenome bei dem Individuum vorherzusagen.

4. Verfahren nach Anspruch 2, wobei die Bestimmung des Methylierungsstatus von mindestens einem CpG in SEQ ID NO: 98 mit der Bestimmung des Methylierungsstatus von mindestens einem CpG kombiniert wird, das in der CpG-reichen Region vorhanden ist, ausgewählt aus der Gruppe bestehend aus: SEQ ID NO: 92, SEQ ID NO: 93, SEQ ID NO: 94, SEQ ID NO: 95, SEQ ID NO: 96, SEQ ID NO: 97, SEQ ID NO: 99 und SEQ ID NO: 100.

5. Verfahren nach Anspruch 1, ferner umfassend ein Bestimmen des Methylierungsstatus von mindestens einem CpG in einer CpG-reichen Region,
wobei die CpG-reiche Region ausgewählt ist aus der Gruppe bestehend aus SEQ ID NO: 27, SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90 und SEQ ID NO: 91 oder einer Sequenz mit einer Sequenzidentität von mindestens 90 % damit.

6. Verfahren nach Anspruch 5, wobei die CpG-reiche Region aus einem kontinuierlichen Abschnitt aus höchstens 300 Basispaaren einer Sequenz besteht, die ausgewählt ist aus der Gruppe bestehend aus SEQ ID NO: 27, SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 oder einer Sequenz, die mindestens 90 % identisch damit ist.

7. Verfahren nach Anspruch 6, wobei die CpG-reiche Region aus einem kontinuierlichen Abschnitt aus höchstens 300 Basispaaren einer Sequenz besteht, die ausgewählt ist aus der Gruppe bestehend aus SEQ ID NO: 1, SEQ ID NO: 8, SEQ ID NO: 10, SEQ ID NO: 11, SEQ ID NO: 18, SEQ ID NO: 22, SEQ ID NO: 23, SEQ ID NO: 25, SEQ ID NO: 32, SEQ ID NO: 33, SEQ ID NO: 35, SEQ ID NO: 36, SEQ ID NO: 38 und SEQ ID NO: 44 oder einer Sequenz, die mindestens 90 % identisch damit ist.

8. Verfahren nach Anspruch 6, wobei mindestens eine CpG-reiche Region mit einem Gen assoziiert ist, das ausgewählt ist aus der Gruppe bestehend aus SCAND3, NPY2R, PRICKLE1, FAM72B, FAM72A, ULBP1, ZNF568, BARHL2, FAT4, FAM72D, EBF2, FAM19A4, NALCN, CD40, MEOX2, ZNF804B, DLX5, FAM133A, NKX2-2, PRMT8, DPP10, SNAP25, KCNA1, ZIC1, SEMA3D, TBCA, PCDH15, mir129-2 und ZNF606.

9. Verfahren nach Anspruch 6, wobei ein Methylierungsstatus von mindestens 2 CpGs in einer CpG-reichen Region bestimmt wird, wobei eine Methylierung von beiden der CpGs ein Hinweis auf das Vorhandensein eines Adenoms des Kolons ist.

10. Verfahren nach einem der vorherigen Ansprüche, wobei der Methylierungsstatus durch multiplexe ligationsabhängige Sondenamplifikation (Multiplex Ligation-dependent Probe Amplification, MLPA) oder ein auf PCR basiertes Verfahren / auf LCR basiertes Verfahren bestimmt wird.

11. Verfahren nach einem der vorherigen Ansprüche, wobei die Probe eine Stuhlprobe oder eine Blutprobe ist.

12. Verfahren nach einem der Ansprüche 1 bis 12, wobei die Probe eine Biopsie des Kolons ist und die Biopsie normale Mukosa und/oder affizierte Mukosa enthält.

## Revendications

1. Procédé de diagnostic comprenant la détermination de l'état de méthylation d'au moins un CpG au sein d'une région riche en CpG dans SEQ ID NO : 98, et dans lequel le procédé comprend l'étape de détermination de l'état de méthylation d'au moins un CpG au sein de ladite région riche en CpG dans l'ADN génomique d'un échantillon d'un individu à risque d'acquérir un adénome dans le côlon ou le rectum, et
dans lequel la méthylation d'au moins un CpG indique la présence d'un adénome du côlon chez ledit individu.

2. Procédé selon la revendication 1, dans lequel la présence de méthylation desdites régions riches en CpG méthylés est utilisée pour prédire l'évolution de la maladie chez ledit individu.

3. Procédé selon la revendication 2, dans lequel la présence de méthylation desdites régions riches en CpG méthylés est utilisée pour prédire la sensibilité à des adénomes de l'individu.

4. Procédé selon la revendication 2, dans lequel la détermination de l'état de méthylation d'au moins un CpG dans SEQ ID NO : 98 est combinée à la détermination de l'état de méthylation d'au moins un CpG présent dans la région riche en CpG choisie dans le groupe constitué de : SEQ ID NO : 92, SEQ ID NO : 93, SEQ ID NO : 94, SEQ ID NO : 95, SEQ ID NO : 96, SEQ ID NO : 97, SEQ ID NO : 99, et SEQ ID NO : 100.

5. Procédé selon la revendication 1 comprenant en outre la détermination de l'état de méthylation d'au moins un CpG au sein d'une région riche en CpG,
dans lequel ladite région riche en CpG est choisie dans le groupe constitué de SEQ ID NO : 27, SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 28, SEQ ID NO : 29, SEQ ID NO : 30, SEQ ID NO : 31, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, SEQ ID NO : 46, SEQ ID NO : 47, SEQ ID NO : 48, SEQ ID NO : 49, SEQ ID NO : 50, SEQ ID NO : 51, SEQ ID NO : 52, SEQ ID NO : 53, SEQ ID NO : 54, SEQ ID NO : 55, SEQ ID NO : 56, SEQ ID NO : 57, SEQ ID NO : 58, SEQ ID NO : 59, SEQ ID NO : 60, SEQ ID NO : 61, SEQ ID NO : 62, SEQ ID NO : 63, SEQ ID NO : 64, SEQ ID NO : 65, SEQ ID NO : 66, SEQ ID NO : 67, SEQ ID NO : 68, SEQ ID NO : 69, SEQ ID NO : 70, SEQ ID NO : 71, SEQ ID NO : 72, SEQ ID NO : 73, SEQ ID NO : 74, SEQ ID NO : 75, SEQ ID NO : 76, SEQ ID NO : 77, SEQ ID NO : 78, SEQ ID NO : 79, SEQ ID NO : 80, SEQ ID NO : 81, SEQ ID NO : 82, SEQ ID NO : 83, SEQ ID NO : 84, SEQ ID NO : 85, SEQ ID NO : 86, SEQ ID NO : 87, SEQ ID NO : 88, SEQ ID NO : 89, SEQ ID NO : 90, et SEQ ID NO : 91 ou une séquence présentant au moins 90 % d'identité de séquence avec celles-ci.

6. Procédé selon la revendication 5 dans lequel la région riche en CpG est constituée d'une suite continue d'au plus 300 paires de bases d'une séquence choisie dans le groupe constitué de SEQ ID NO : 27, SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 28, SEQ ID NO : 29, SEQ ID NO : 30, SEQ ID NO : 31, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, ou une séquence au moins identique à 90 % à celles-ci.

7. Procédé selon la revendication 6, dans lequel la région riche en CpG est constituée d'une suite continue d'au plus 300 paires de bases d'une séquence choisie dans le groupe constitué de SEQ ID NO : 1, SEQ ID NO : 8, SEQ ID NO : 10, SEQ ID NO : 11, SEQ ID NO : 18, SEQ ID NO : 22, SEQ ID NO : 23, SEQ ID NO : 25, SEQ ID NO : 32, SEQ ID NO : 33, SEQ ID NO : 35, SEQ ID NO : 36, SEQ ID NO : 38, et SEQ ID NO : 44 ou une séquence au moins identique à 90 % à celles-ci.

8. Procédé selon la revendication 6, dans lequel au moins une région riche en CpG est associée à un gène choisi dans le groupe constitué de SCAND3, NPY2R, PRICKLE1, FAM72B, FAM72A, ULBP1, ZNF568, BARHL2, FAT4, FAM72D, EBF2, FAM19A4, NALCN, CD40, MEOX2, ZNF804B, DLX5, FAM133A, NKX2-2, PRMT8, DPP10, SNAP25, KCNA1, ZIC1, SEMA3D, TBCA, PCDH15, mir129- 2 et ZNF606.

9. Procédé selon la revendication 6, dans lequel l'état de méthylation d'au moins 2 CpG au sein d'une région riche en CpG est déterminé, dans lequel la méthylation des deux dits CpG indique la présence d'un adénome du côlon.

10. Procédé selon l'une quelconque des revendications précédentes, dans lequel l'état de méthylation est déterminé par une amplification multiplex de sonde nucléique dépendant des ligatures (MLPA) ou un procédé basé sur la PCR/un procédé basé sur la LCR.

11. Procédé selon l'une quelconque des revendications précédentes, dans lequel l'échantillon est un échantillon fécal ou un échantillon sanguin.

12. Procédé selon l'une quelconque des revendications 1 à 12, dans lequel l'échantillon est une biopsie du côlon, ladite biopsie contenant de la muqueuse normale et/ou de la muqueuse affectée.
